(19) Europäisches Patentamt
European Patent Office
Office européen des brevets

(11) Publication number: 0 190 900 A2

# (12) EUROPEAN PATENT APPLICATION

(21) Application number: 86300690.4

(22) Date of filing: 31.01.86

(51) Int. Cl.[4]: C 07 D 501/36, A 61 K 31/545

(30) Priority: 31.01.85 JP 18067/85
06.08.85 JP 173746/85
25.12.85 JP 295383/85

(43) Date of publication of application: 13.08.86 Bulletin 86/33

(84) Designated Contracting States: AT BE CH DE FR GB IT LI LU NL SE

(71) Applicant: YAMANOUCHI PHARMACEUTICAL CO. LTD.
No. 5-1 Nihonbashi-Honcho, 2-chome Chuo-ku
Tokyo(JP)

(72) Inventor: Shibanuma, Tadao
No. 596-11, Oyaguchi
Urawa-shi Saitama(JP)

(72) Inventor: Yamazaki, Atsuki
No. 7-8-506, Shimura 2-chome Itabashi-ku
Tokyo(JP)

(72) Inventor: Koda, Akio
No. 5-3, Hibarigaoka Kita 4-chome
Hoya-shi Tokyo(JP)

(72) Inventor: Maeda, Tetsuya
No. 14-6, Yotsuya, 3-chome
Urawa-shi Saitama(JP)

(72) Inventor: Nakano, Kohji
No. 1086-1, Oaza Sanagaya Shiraoka-cho
Minamisaitama-gun Saitama(JP)

(72) Inventor: Nagano, Noriaki
13-1, Ayase
Hasuda-shi Saitama(JP)

(72) Inventor: Murakami, Yukiyasu
306-3, Omaki
Urawa-shi Saitama(JP)

(72) Inventor: Hara, Ryuichiro
No. 19-11, Honcho 3-chome Nakano-ku
Tokyo(JP)

(72) Inventor: Takahashi, Takumi
16-1m Hasune 3-chome Itabashi-ku
Tokyo(JP)

(74) Representative: Geering, Keith Edwin et al,
REDDIE & GROSE 16 Theobalds Road
London WC1X 8PL(GB)

## (54) Cephalosporin compounds, their production, and pharmaceutical compositions containing them.

(57) Novel cephalosporin compounds of the formula and salts thereof, and some production methods thereof

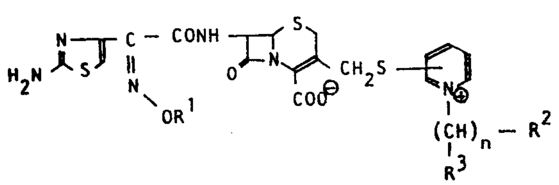

wherein $R^1$ represents a lower alkyl group which is unsubsitutted or substituted by a cyano group or a halogen atom, $R^2$ represents a hydroxy lower alkyl group, a cyano group, a lower alkylthio group, a lower alkenyl group substitutable by carboxy, a lower alkinyl group, an imidazolyl group, a $C_3$ to $C_8$ alicyclic group, a 2–oxo–1, 3–dioxol–5–yl group, a 2–carboxy–I, 3–dithiolan–2–yl group, $-COR^A$ (wherein $R^A$ is an amino group, a pyridyl group, a carboxyl group, a $C_3$ to $C_8$ alicyclic group, a lower alkyl group which is unsubstituted or substituted by a halogen atom), or

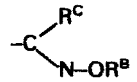 (wherein $R^B$ represents a lower alkyl group which is unsubstituted or substitued by a carboxyl group, $R^C$ represents a carboxyl group or a lower alkyl group), n represents an integer of 1 to 3; $R^3$ represents a hydrogen atom or a lower alkyl group. These compounds have excellent antibacterial activities and are useful for the prevention and treatment of bacterial infections. The preparation and the use of these compounds as antibacterial agents is described.

EP 0 190 900 A2

Croydon Printing Company Ltd.

CEPHALOSPORIN COMPOUNDS, THEIR PRODUCTION, AND PHARMACEUTICAL COMPOSITIONS CONTAINING THEM

This invention relates to antibacterial caphalosporin compounds showing antibacterial acitivy against various pathogens, the production of these compounds, medical compositions containing them,

The compounds of this invention are those of the general formula (I) and salts thereof:

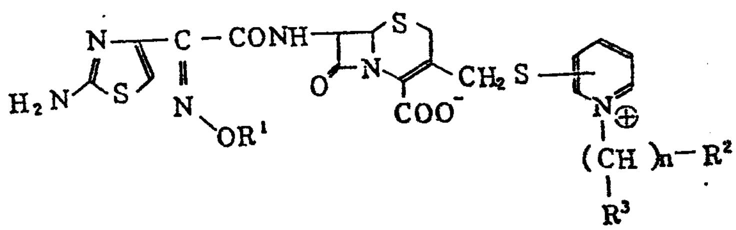

wherein $R^1$ represents a lower alkyl group which may have at least one substituent selected from cyano and halogen moieties; $R^2$ represents a hydroxy lower alkyl group, a cyano group, a lower alkylthio group, a lower alkenyl group substitutable by carboxy, a lower alkinyl group, an imidazolyl group, a $C_3$ to $C_6$ alicyclic group, a 2-oxo-1,3-dioxol-5-yl group, a 2-carboxy-1,3-dithiolan-2-yl group, $-COR^A$ (wherein $R^A$ is an amino group, a pyridyl group, a carboxyl group, a $C_3$ to $C_6$ alicyclic group, or a lower alkyl group which is unsubstituted or substituted by halogen),or

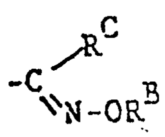

(wherein $R^B$ represents a lower alkyl group which is unsubstituted or substituted by carboxyl, and $R^C$ represents a carboxyl group or a lower alkyl group); n represents 1, 2 or 3; and $R^3$ represents a hydrogen atom or a lower alkyl group.

The processes of this invention include processes A to D below.

Process A

A method of preparing a compound of formula (I) as defined above or a salt thereof

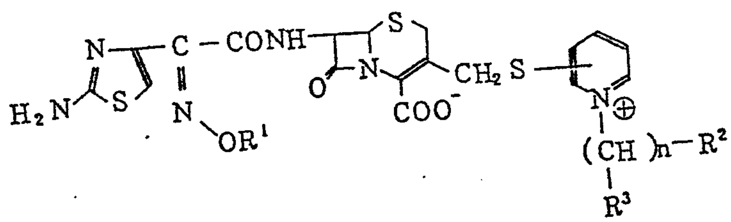

(I)

which comprises reacting a compound of the formula

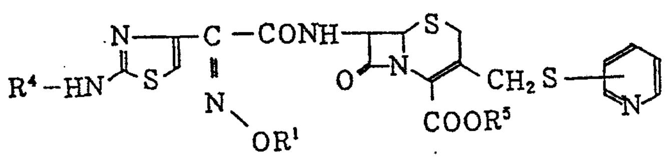

(wherein $R^4$ is a hydrogen atom or a protective group for an amino group and $R^5$ is a hydrogen atom or a protective group for a carboxyl group) with a compound of the formula

$$X-\overset{\displaystyle R^3}{\overset{|}{(CH)}}_n-R^{20}$$

(wherein $R^{20}$ is $R^2$ as defined above in which any carboxy group may be protected) and if necessary,

releasing any protective group(s) and/or converting to a salt.

Process B

A method of preparing a compound of formula (I) as defined above or a salt thereof which comprises reacting a compound of the formula

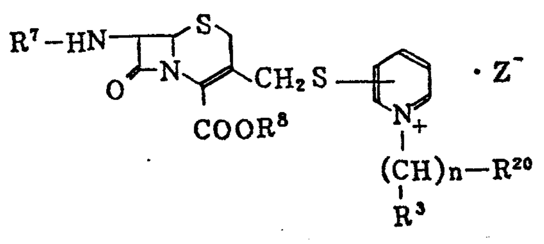

(wherein $R^{20}$ and n are as defined above, $R^7$ is a hydrogen atom or an tri- lower alkylsilyl group; $Z^-$ represents an anion; and $R^8$ is a hydrogen atom or a protective group for a carboxyl group except that-$COOR^8$ may be $-COO^-$ in which case $Z^-$ is absent)

with a compound of the formula

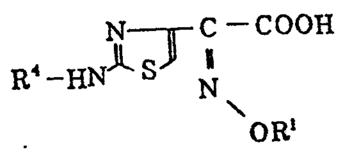

(wherein $R^1$ and $R^4$ are as defined above) or a reactive derivative thereof, and if necessary, releasing any protective group(s) and/or converting to a salt.

Process C

A method of preparing a compound of formula (I) as defined above or a salt thereof which comprises reacting a compound of the formula

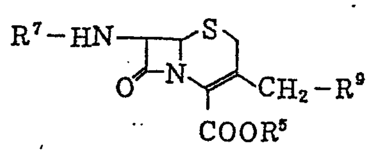

(wherein $R^5$ and $R^7$ are as defined above and $R^9$ is a halogen atom or a lower acyloxy group)

with a compound of the formula

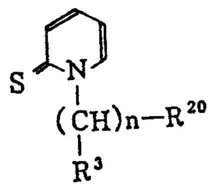
,
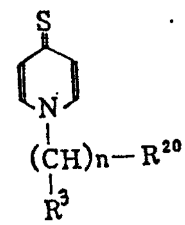
, or
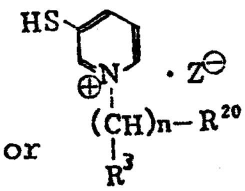

(wherein $R^{20}$, $R^3$ and n are as defined above)

to form a compound of the formula

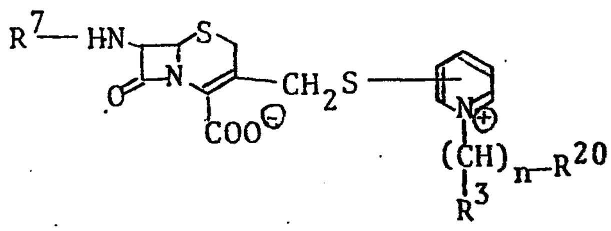

and then reacting the formed compound with a compound of the formula

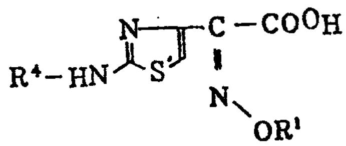

(wherein $R^1$ and $R^4$ are as defined above)

or a reactive derivative thereof, and if necessary, releasing any protective group(s) and /or converting to a salt.

Process D

A method of preparing a compound of formula (I) as defined above or a salt thereof which comprises reacting a compound of the formula

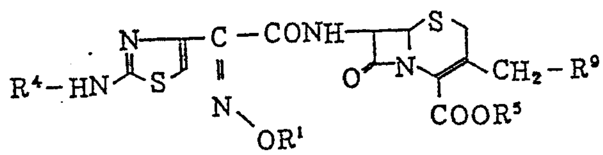

(wherein $R^1$, $R^4$, $R^5$ and $R^9$ are as defined above) with a compound of the formula

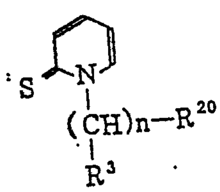 , 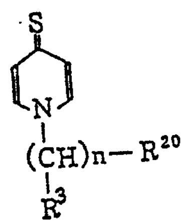 or 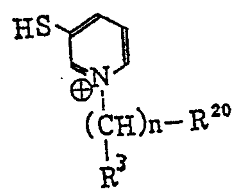

(wherein $R^3$, $R^{20}$ and n are as defined above) and then, if necessary, releasing any protective group(s) and/or converting to a salt.

Many cephalosporin compounds are known and somewhat related compounds are disclosed in German Publication Nos. 2921332 and 2946415, etc.

Compounds of this invention show strong antibacterial activity against a wide range of pathogens including many important gram positive and negative pathogens such as Pseudomonas aeruginosa, and thus are useful for medicaments (especially antibacterial agents) for use in a treatment of bacterial infection diseases in human beings and animals, as additives for feeds, and as preservatives. Compounds of this invention have low toxicity and hence are more advantageous than other known cephalosporin compounds.

The term "lower alkyl" in the definitions herein means a straight or branched carbon chain alkyl having 1 to 5 carbon atoms.

The term "lower" in the definitions for alkenyl or alkinyl means a straight or branched carbon chain having 2 to 5 carbon atoms.

The compounds (I) with an iminoether-type oxime and 2-substituted thiazol group include geometrical isomers and tautomers; this invention includes all these syn-form and anti-form geometrical isomers and mutual tautomers, alone and in any combination.

Examples of the unsubstituted "lower alkyl group" are methyl, ethyl, propyl, iso-propyl, butyl, iso-butyl, sec-butyl, tert-butyl, pentyl (amyl), iso-pentyl, tert-pentyl, neopentyl, 1-methylbutyl, 2-methylbutyl, 1,2-dimethylpropyl, 1-ethylpropyl, and pentyl groups.

The $R^1$ lower alkyl group may be substituted by cyano or halogen at any position(s) of the alkyl chain, e.g. cyanomethyl, cyanoethyl, fluoromethyl, fluoroethyl, chloroethyl, trifluoromethyl, etc.

Examples of the lower alkyl group which is substituted by halogen (in the definition of $R^A$) are fluoromethyl, fluoroethyl, chloroethyl groups. Examples of the lower alkyl group which is substituted by carboxy (in the definition of $R^B$) are carboxymethyl, 2-carboxyethyl, 1-carboxyethyl, 1-carboxy-1-methylethyl groups. Examples of the hydroxy lower alkyl groups are hydroxymethyl, 1-hydroxyethyl, 2-hydroxyethyl, groups. Examples of the carboxy lower alkenyl group are 1-carboxyvinyl, 2-carboxyvinyl, 1-carboxyallyl, 2-carboxyallyl, 3-carboxyallyl, 1-carboxy-1-propenyl, 2-carboxy-1-propenyl, 3-carboxy-1-propenyl, 2-carboxy-1-methylvinyl, 1-carboxy-1-butenyl, 2-carboxy-2-butenyl, 3-carboxy-3-butenyl, 4-carboxy-3-butenyl, 2-carboxy-1-methyl-2-propenyl, 4-carboxy-4- pentenyl groups. Examples of the lower alkinyl group are ethinyl, 1-propinyl, 1-butinyl, 2-butinyl, 1-pentinyl, 2-pentinyl. Examples of the lower alkylthio group are methylthio, ethylthio,propylthio, iso-propylthio, butylthio, iso-butylthio, sec-butylthio, tert-butylthio, pentylthio, iso-pentylthio, tert-pentylthio, neopentylthio groups. Examples of a $C_3$ to $C_6$ alicyclic group are cyclopropyl, cyclobutyl, cyclopentyl, cyclohexyl groups.

The compounds (I) have the characteristics of an α-(2-amino-4-thiazolyl)-α-(loweralkoxyimino)acetamido group at the 7-position of the cephalosporin nucleus and a 1-substituted-pyridiniothiomethyl group at the 3-position of the nucleus. Antibacterial activities (minimum effective inhibitory concentrations) of compounds of formula I (taken from the following Examples) are shown in the following Table.

Table (minimum effective inhibitory concentration μg/ml)

| Example No. / Strain | 1 | 4 | 9 | 12 | 15 | 17 | 20 | Cefmenoxime |
|---|---|---|---|---|---|---|---|---|
| S. aureus FDA 209P JC-1 | 0.2 | 0.2 | 0.1 | 0.2 | 0.2 | 0.2 | 0.2 | 1.56 |
| S. epidermidis IID 866 | 0.1 | 0.1 | 0.1 | 0.1 | 0.1 | 0.2 | 0.1 | 0.39 |
| E. coli D-1 | 0.013 | ≦0.006 | ≦0.006 | 0.025 | 0.013 | 0.013 | ≦0.006 | 0.025 |
| K. pneumoniae ATCC 10031 | 0.013 | ≦0.006 | ≦0.006 | 0.013 | 0.013 | 0.013 | ≦0.006 | ≦0.006 |
| P. aeruginosa NCTC 10490 | 0.39 | 0.2 | 0.39 | 0.39 | 0.39 | 0.78 | 0.39 | 0.2 |

The salts of the compounds of this invention of formula I are the pharmacologically acceptable nontoxic salts of the compounds such as nontoxic acid addition salts or base addition salts. Examples of the acid addition salts are pyridinium salts of the formula:

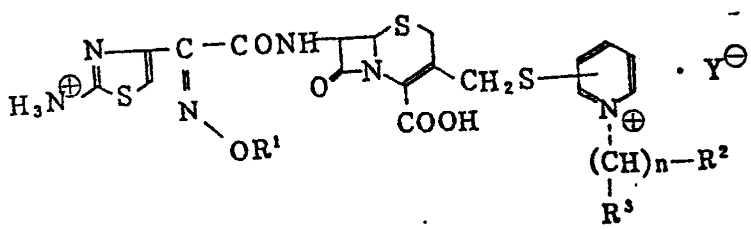

wherein $Y^{\ominus}$ represents an inorganic anion such as nitrate anion ($NO_3^-$), halogen anion ($Cl^-$, $I^-$, etc.) or a sulfonic acid anion ($HSO_4^-$, $SO_4^{--}$, etc.); or an organic anion such as an acetate anion ($CH_3COO^-$), a fumarate anion ($HOOC-CH:CH-COO^-$), a citrate anion ($HOOC-CH_2-C(OH)(COOH)-CH_2COO^-$) or a benzenesulfonate anion ($C_6H_5SO_3^-$).

Examples of the base addition salts are salts of the following formula:

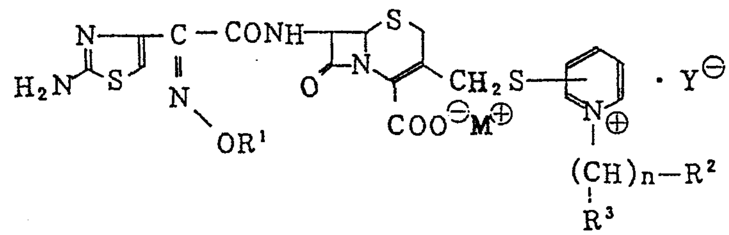

wherein $M^{\oplus}$ represents an alkali metal cation such as $Na^+$ or $K^+$; $NH_4^+$; or an organic base cation such as 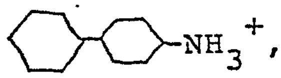, 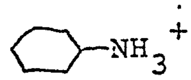, $(CH_3)_3NH_3^+$, $(C_2H_5)_3NH_3^+$, $HO-CH_2CH_2-NH_3^+$, ornithinium, or lysinium.

Among the above salts, nitrates are preferred.

Antibacterial medicaments containing compounds according to the invention may be prepared by conventional methods using conventional carriers or excipients. They may for example be administered parenterally by intravenous or intramuscular injection; orally as tablets, pills, capsules, granules; as liquid preparations; or as suppositories. The appropriate dose is determined in each case considering factors such as the symptom, age and sex of the patient. For an adult a daily total of 250 to 3,000 mg is usually administered in one to four doses.

Some of the compounds of this invention have low toxicity (acute toxicity).

The compound of Example 4 has, in particular, low toxicity.

200 mg of /the compound of Example 4 was dissolved in 2 ml of distilled water and after adjusting the pH of the solution to about 7 by addition of sodium hydrogen carbonate to form an injection solution, the solution was intravenously injected into mice (body weights: 26-28 g; ICR♂ 5W) over a period of 4 minutes at a rate of 1 ml/kg. There were no lethal cases for administration doses below 4.0 g/kg.

The compounds of this invention can be produced by various processes; typical production processes are explained hereinafter.

Process A:

A compound of general formula I can be produced by reacting a compound of the following general formula II with a compound of the following general formula III, and then, if necessary, releasing any protective group.

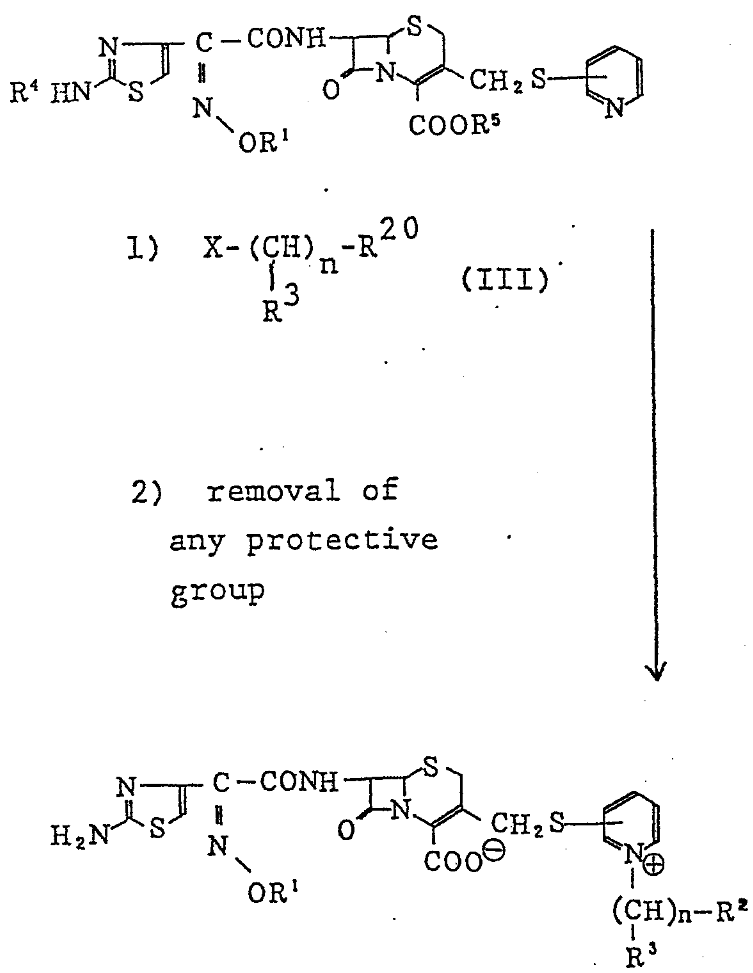


(II)

(I)

Process B and Process C

A compound of general formula I can be produced by reacting a compound of the following general formula VI with N,O-bis(tri-loweralkylsilyl)trifluoroacetamide and a compound of the following general formula VII and then reacting the formed compound with a compound of the following general formula V and then, if necessary, releasing any protective group.

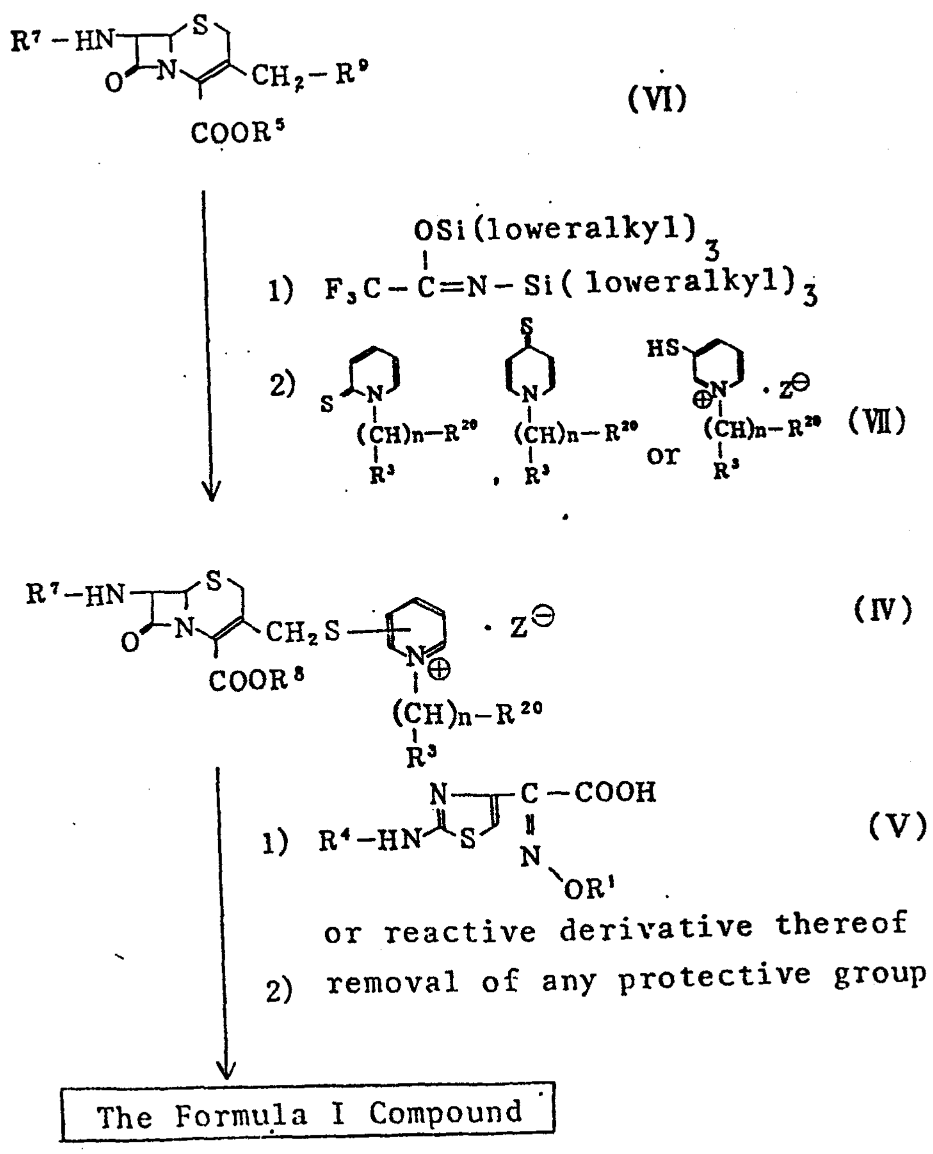


(VI)

(VII)

(IV)

(V)

Process D

A compound of general formula I can be produced by reacting a compound of the following general formula VIII with a compound the formula VII and then, if necessary, releasing any protective group.

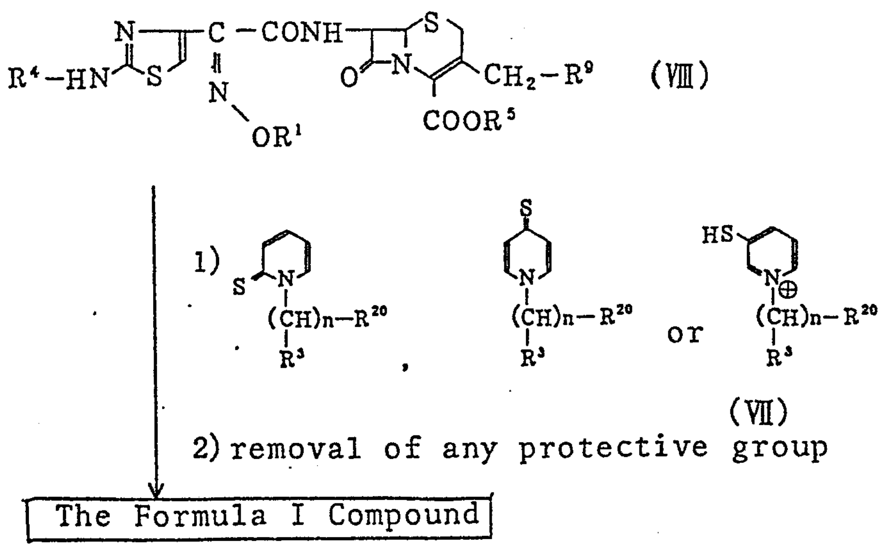


(VIII)

, or

(VII)

In the above formulae I to VIII

$R^1$, $R^2$, $R^{20}$, $R^3$, $R^4$, $R^5$, $R^6$, $R^7$, $R^8$, $R^9$, X, $Z^-$ and n are as previously defined.

Examples of the halogen atoms in the definitions of X and $R^9$ are a chlorine atom, a bromine atom, an iodine atom. Examples of $R^6$ in the definition of $-OSO_2-R^6$ (sulfonate) are methyl, ethyl (for lower alkyl), phenyl, tolyl (for aryl), trifluoromethyl. Examples of acyloxy in the definition of $R^9$ are acetoacetoxy, acetoxy, propionyloxy, iso-propionyloxy, butyryloxy, iso-butyryloxy, pentyloxy. Examples of an amino-protecting group in the definitions of $R^4$ are an acyl group such as a formyl, acetyl, propionyl, tert-butoxycarbonyl, methoxyacetyl, methoxypropionyl, benzyloxycarbonyl, or p-nitrobenzyloxycarbonyl group; an aralkyl group such as a benzyl, benzhydryl, (diphenylmethyl), or trityl group; and a trilowerlakylsilyl group such as trimethylsilyl; thus the amino-protecting group may be one usually used in the field of peptide chemistry. Examples of a protecting group for a carboxy or carboxyloweralkenyl group in the definitions of $R^5$ and $R^{20}$ are those which can be released easily under mild conditions, e.g. tri(lower)alkylsilyl such as trimethylsilyl, benzhydryl, $\beta$-methylsulfonylethyl, phenacyl, p-methoxybenzyl, tert-butyl, and p-nitrobenzyl groups.

Practical reaction conditions of Process A:

It is preferred that the reaction is performed in organic solvents which do not take part in the reaction. Examples of such solvents are dichloromethane, dimethylsulfoxide, dimethylformamide, benzene, toluene, chloroform,

ethyl acetate, ether, tetrahydrofuran, dioxane.

Equimolar- or excess amount of either of the starting materials (Compounds II and/or III) may be used in the reaction. The reaction proceeds easily at room temperature or under cooling.

The removal of protecting groups from the reaction product is easily performed. For example, when the group is triloweralkylsilyl, its removal is easily performed by contact with water; when the group is benzhydryl, trityl, tert-butyl, or formyl, its removal is easily performed by treatment with acid such as formic acid, trifluoroacetic acid, trifluoroacetic acid-anisole mixture, hydrobromic acid-acetic acid mixture; hydrochloric acid-dioxane mixture.

Practical reaction conditions of Process B and Process C:

In these reaction schemes, Process C means the reaction course: formula VI compound ⟶ formula IV compound ⟶ formula I compound; Process B means only its second step: formula IV compound ⟶ formula I compound.

For example, a formula VI compound in the case of $R^5$ and $R^7$ being hydrogen and $R^9$ being acetoxy (or halogen), or in the case of 7-amino being protected, is reacted with a formula VII compound (namely, 1-substituted-2-(or 4-)-thiopyridone or 1-substituted-3-mercapto-pyridinium) in a solvent which does not take part in the reaction.

For promoting the reaction, a catalyst of a salt (e.g. KI, No.I), an acid such as a Lewis acid (e.g. boron trifluororide, ether), chlorosulfonic acid, fluorosulfonic acid, trifluoromethanesulfonic acid may be preferrably added to the reaction system. In the case of an acid being added for promoting the reaction, the formed compound is obtained as the corresponding salt, which is easily converted to the free compound by conventional methods e.g. by subjecting the salt to column chromatography, e.g. ion exchange column chromatography on Diaion HP-20 ((Mitsubishi Chemical Co., Japan)), etc. This first step reaction (VI) → (IV) may be performed by reacting 7-aminocephalosporanic acid or its derivative in the case of $R^9$ being halogen (a formula VI compound) with N,O-bis(triloweralkylsilyl)trifluoroacetamide to protect an amino group and/or a carboxy group with silyl type group(s), and then reacting the resulting protected compound with a formula VII compound. The second step (Process B) can be performed by reacting the formula IV compound (obtained in the first step) with a formula V compound (namely, α-(2-substituted-4-thiazolyl)-α-substitutediminoacetic acid or its reactive derivative). The compound IV may be an inner salt (that is, $-COOR^8 = COO^{\ominus}$) or, in the case of $R^8$ being a protective group, may be a salt in the case of $Z^{\ominus}$ being an anion. Reaction of compounds V and IV is usually performed in a solvent under cooling or at room temperature. Any solvent which does not take part in the reaction may be used and

examples of the solvents are water and organic solvents such as acetone, dioxane, ether, tetrahydrofuran, ethyl-methylketone, chloroform, dichloroethane, ethyl acetate, ethyl formate, dimethylformamide, dimethylsulfoxide; these solvents may be used alone or in appropriate combination. The compound V may be a free carboxylic acid or a reactive derivative thereof. Suitable examples of such reactive derivative are active esters (e.g. benzotriazole ester), mixed acid anhydrides, acid anhydrides, acid halides, active amides, and acid azides. When using a free carboxylic acid V, it is preferred to use a condensing agent such as N,N'-dicyclohexylcarbodiimide or N,N'-diethylcarbodiimide. When using a reactive derivative of a carboxylic acid it may be preferred for smooth reaction to operate in the presence of a base. Examples of such a base are inorganic bases such as sodium hydrogencarbonate potassium hydrogencarbonate, sodium carbonate, and potassium carbonate; and organic bases such as trimethylamine, triethylamine and dimethylaniline, and pyridine.

Practical reaction conditions of Process D:

The reaction is usually performed by stirring compound (VIII) or a salt thereof and compound (VII) in water, phosphoric acid buffer, or other inert solvent or a mixture thereof.
Examples of inert solvent for this purpose are alcohols such as methanol, ethanol; amides such as dimethylformamide, acetamide; and acetones and acetonitriles. For promoting the reaction, a catalyst such as potassium iodide, potassium bromide, sodium bromide, or potassium thiocyanate may be added to the reaction system in an excess amount. The reaction proceeds easily at room temperature or under heating.

0190900

Compounds shown by the following formula are according to the invention and can be obtained by the first step of Process C:

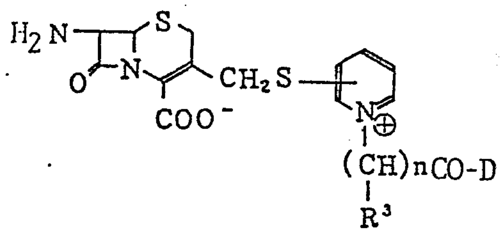

wherein $R^3$ is as defined above, n is 1, 2 or 3, and D is a lower alkyl group.

A salt of the formula I compound can be produced by performing a foregoing production process using a salt of the starting compound, or by applying a salt-forming reaction to the free formula I compound.

The formula I compounds and salts can be separated and purified in conventional manner, such as extraction with organic solvent, crystallization, or column chromatography.

0190900

By using the before-mentioned processes, the following compounds were obtained.

(1) (Z)-7-[α-(2-amino-4-thiazolyl)-α-(methoxyimino)-acetamido]-3-(1-cyanomethyl-4-pyridinio)thiomethyl-Δ$^3$-cephem-4-carboxylate.

(2) (Z)-7-[α-(2-amino-4-thiazolyl)-α-(methoxyimino)-acetamido]-3-[1-(2-carboxylate-2-propenyl)-4-pyridinio]-thiomethyl-Δ$^3$-cephem-4-carboxylate.

(3) (Z)-7-[α-(2-amino-4-thiazolyl)-α-(methoxyimino)-acetamido]-3-[1-(methylthiomethyl)-4-pyridinio]thio-methyl-Δ$^3$-cephem-4-carboxylate.

(4) (Z)-7-[α-(2-amino-4-thiazolyl)-α-(methoxyimino)-acetamido]-3-[1-acetonyl-4-pyridinio]thiomethyl-Δ$^3$-cephem-4-carboxylate.

(5) (Z)-7-[α-(2-amino-4-thiazolyl)-α-(methoxyimino)-acetamido]-3-(1-cyclopropylmethyl-4-pyridinio)thio-methyl-Δ$^3$-cephem-4-carboxylate.

(6) (Z)-7-[α-(2-amino-4-thiazolyl)-α-(methoxyimino)-acetamido]-3- [ 1-acetonyl-4-pyridinio]thiomethyl-Δ$^3$-cephem-4-carboxylate.

(7) (Z)-7-[α-(2-amino-4-thiazolyl)-α-(methoxyimino)-acetamido]-3-[1-(2-oxobutyl)-4-pyridinio]thiomethyl-Δ$^3$-cephem-4-carboxylate.

(8) (Z)-7-[α-(2-amino-4-thiazolyl)-α-(methoxyimino)-acetamido]-3-(1-(1-methyl-2-oxopropyl)-4-pyridinio)-thiomethyl-Δ$^3$-cephem-4-carboxylate.

(9) (Z)-7-[α-(2-amino-4-thiazolyl)-α-(methoxyimino)-acetamido]-3-[1-propargyl-4-pyridinio]thiomethyl-Δ$^3$-cephem-4-carboxylate.

(10) (Z)-7-[α-(2-amino-4-thiazolyl)-α-(methoxyimino)-acetamido]-3-[1-carbamoylmethyl-4-pyridinio] thiomethyl-$\Delta^3$-cephem-4-carboxylate.

(11) (Z)-7-[α-(2-amino-4-thiazolyl)-α-(cyanomethoxy-imino)acetamido]-3-(1-acetonyl-4-pyridinio)thiomethyl-$\Delta^3$-cephem-4-carboxylate.

(12) (Z)-7-[α-(2- amino-4-thiazolyl)-α-(2-fluoro-ethoxyimino)acetamido]-3-(1-acetonyl-4-pyridinio)thio-methyl-$\Delta^3$-cephem-4-carboxylate.

(13) (Z)-7-[α-(2-amino-4-thiazolyl)-α-(methoxyimino)-acetamido]-3-(1-(2-pyridyl-2-oxoethyl)-4-pyridinio)-thiomethyl-$\Delta^3$-cephem-4-carboxylate.

(14) (Z)-7- [α-(2-amino-4-thiazolyl)-α-(methoxyimino)-acetamido-3-(1-(2-cyclopropyl-2-oxo)ethyl-4-pyridinio)-thiomethyl-$\Delta^3$-cephem-4-carboxylate.

(15) (Z)-7-[α-(2-amino-4-thiazolyl)-α-(methoxyimino)-acetamido-3-[1-(4-imidazolyl)methyl-4-pyridinio]-thio-methyl-$\Delta^3$-cephem-4-carboxylate.

(16) (Z)-7-[α-(2-amino-4-thiazolyl)-α-(methoxyimino)-acetamido]-3-[1-(3-fluoro-2-oxopropyl)-4-pyridinio)-thiomethyl-$\Delta^3$-cephem-4-carboxylate.

(17) (Z)-7-[α-(2-amino-4-thiazolyl)-α-(methoxyimino)-acetamido]-3-[1-(2-methoxyiminopropyl)-4-pyridiniothio-methyl]-$\Delta^3$-cephem-4-carboxylate.

(18) (Z)-7-[α-(2-amino-4-thiazolyl)-α-(methoxyimino)-acetamido]-3-[1-(5-methyl-2-oxo-1,3-dioxolene-4-yl)methyl-4-pyridiniothiomethyl]-$\Delta^3$-cephem-4-carboxylate.

(19) (Z)-7-[α-(2-amino-4-thiazolyl)-α-(methoxyimino)-acetamido]-3-[1-(3-methyl-2-oxobutyl)-4-pyridinio]thio-methyl-$\Delta^3$-cephem-4-carboxylate.

(20) (Z)-7-[α-(2-amino-4-thiazolyl)-α-(methoxyimino)-acetamido]-3-[1-(2-oxopentyl)-4-pyridinio]thiomethyl-$\Delta^3$-cephem-4-carboxylate.

(21) (Z)-7-[α-(2-amino-4-thiazolyl)-α-(methoxyimino)-acetamido]-3-[1-(2-carboxy-2-oxoethyl)-4-pyridinio-thiomethyl]-$\Delta^3$-cephem-4-carboxylate.

(22) (Z)-7-[α-(2-amino-4-thiazolyl)-α-(methoxyimino)-acetamido]-3-[[1-(2-(1-carboxy-1-methylethoxyimino)-propyl]-4-pyridiniothiomethyl]-$\Delta^3$-cephem-4-carboxylate.

(23) (Z)-7-[α-(2-amino-4-thiazolyl)-α-(methoxyimino)-acetamido]-3-[1-acetonyl-4-pyridinio]thiomethyl-$\Delta^3$-cephem-4-carboxylate ($2HNO_3$ salt)

(24) (Z)-7-[α-(2-amino-4-thiazolyl)-α-(methoxyimino)-acetamido]-3-[1-acetonyl-4-pyridinio]thiomethyl-$\Delta^3$-cephem-4-carboxylate. (Process A)

(25) (Z)-7-[α-(2-amino-4-thiazolyl)-α-(methoxyimino)-acetamido]-3-[1-acetonyl-4-pyridinio]thiomethyl-$\Delta^3$-cephem-4-carboxylate. (Process B)

(26) (Z)-7-[α-(2-amino-4-thiazolyl)-α-(methoxyimino)-acetamido]-3-[1-acetonyl-4-pyridinio]thiomethyl-$\Delta^3$-cephem-4-carboxylate (nitrate)

(27) (Z)-7-[α-(2-amino-4-thiazolyl)-α-(methoxyimino)-acetamido]-3-[1-acetonyl-4-pyridinio]thiomethyl-$\Delta^3$-cephem-4-carboxylate (Process C)

0190900

(28) (Z)-7-[α-(2-amino-4-thiazolyl)-α-(methoxyimino)-acetamido]-3-[1-acetonyl-4-pyridinio]thiomethyl-$\Delta^3$-cephem-4-carboxylate ( sulfate)

(29) (Z)-7-[α-(2- amino-4-thiazolyl)-α-(methoxyimino)-acetamido]-3-[1-acetonyl-4-pyridinio] thiomethyl-$\Delta^3$-cephem-4-carboxylate. (Process D)

(30) (Z)-7-[α-(2-amino-4-thiazolyl)-α-(methoxyimino)-acetamido]-3-[1-(2-hydroxypropyl)-4-pyridinio]thiomethyl-$\Delta^3$-cephem-4-carboxylate.

(31) (Z)-7-[α-(2-amino-4-thiazolyl)-α-(methoxyimino)-acetamido]-3-[1-[(2-carboxy-1,3-dithiolane-2-yl)methyl]-4-pyridiniomethyl]-$\Delta^3$-cephem-4-carboxylate.

The invention is illustrated by the following Examples (according to the invention) and Reference Examples.

Below, NMR, IR, and mp. are abbreviations for nuclear magnetic resonance spectrum, infrared spectrum, and melting point.

Reference Example 1

(raw material for Examples 1 to 5, 7 to 10 and 13 to 22)

(a)

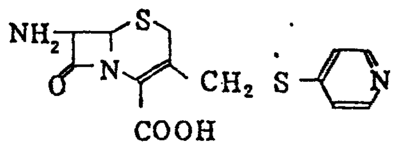

In 200 ml of water was suspended 13.6 g of 7-aminocephalosporanic acid and, 5.55 g of 4-mercaptopyridine and 8.4 g of hydrogen sodium carbonate were added to the suspension. After the resulting solution was stirred at an inner temperature of 55 to 60°C for 5 hours, the reaction mixture was cooled to 10°C and the pH was adjusted to 1 - 2 with 1N hydrochloric acid. The precipitate was filtered. After washing the precipitate with water, it was dried to obtain 15.9 g of 7-amino-3-(4-pyridinyl)thiomethyl-$\Delta^3$-cephem-4-carboxylic acid.

NMR (DMSO - $d_6$)

δ : ppm 3.66 (2H, q, 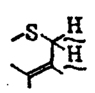 )

4.30 (2H, q, 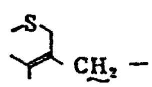 )

4.98, 5.13 ( 1H, d, 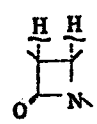 )

7.41 (2H, d, 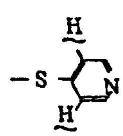 )

8.44 (2H, d, 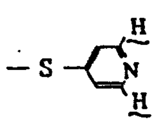 )

(b-i)



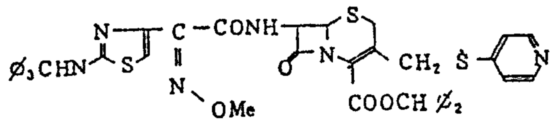

In 50 ml of dichloromethane was suspended 1.615 g of 7-amino-3-(4-pyridinyl)thiomethyl-$\Delta^3$-cephem-4-carboxylic acid and, 5.14 g of O,N-bis(trimethylsilyl)trifluoroacetamide was added to the suspension. The mixture was stirred at room temperature for 3 hours to form a homogeneous solution. The solution was cooled to -50°C and 1.975 g of pyridine was added thereto (Liquid A).

On the other hand, 2.22 g of (Z)-α-(2-tritylamino-4-thiazolyl)-α-(methoxyimino)acetic acid was suspended in 10 ml of dichloromethane. Under ice cooling, 1.15 g of phosphorus pentachloride was added to the suspension. The mixture was stirred for 15 minutes (Liquid B).

After dropwise adding Liquid B to Liquid A, the temperature was elevated to -15°C over 15 minutes and the mixture was stirred at this temperature for 10 minutes. To the solution were added 20 ml of 1N hydrochloric acid, 20 ml of water and 10 ml of tetrahydrofuran. After the mixture was stirred for 10 minutes under ice cooling, dichloromethane and tetrahydrofuran were removed from the reaction mixture by distillation under reduced pressure. To the residue 100 ml of water was added. The resulting solid was filtered, washed with water and dried to obtain 2.88 g of crude (Z)-7-[α-(2-tritylamino-4-thiazolyl)-α-(methoxyimino)-

acetamido]-3-(4-pyridinyl)thiomethyl-$\Delta^3$-cephem-4-carboxylic acid.

(b-ii) In 20 ml of dichloromethane was dissolved 2.88 g of crude (Z)-7-[α-(2-tritylamino-4-thiazolyl)-α-(methoxy-imino)acetamido]-3-(4-pyridinyl)thiomethyl-$\Delta^3$-cephem-4-carboxylic acid. Under ice cooling 896 mg of diphenyldiazomethane was added to the solution. After stirring at room temperature for 1 hour, the reaction mixture was concentrated. The residue was subjected to silica gel chromatography and eluted firstly with benzene and then with benzene : ethyl acetate (1:1) to obtain 0.9 g of diphenylmethyl (Z)-7-[α-(2-tritylamino-4-thiazolyl)-α-(methoxyimino)acetamido]-3-(4-pyridinyl)thiomethyl-$\Delta^3$-cephem-4-carboxylate.

NMR (DMSO - $d_6$)

δ : ppm 3.63 (2H, q, 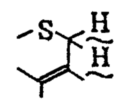 )

3.81 (3H, s, 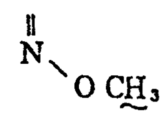 )

4.14 (2H, q, 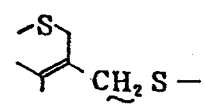 )

5.21 (1H, d, 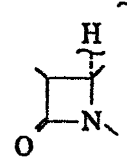 )

5.76 (1H, dd, 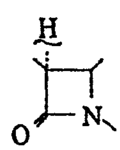 )

6.75 (1H, s, 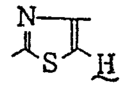 )

6.97 (1H, s, $-COOCH\phi_2$ )

7.04 ~ 7.64 (27H, m, 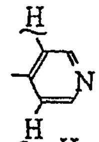 +

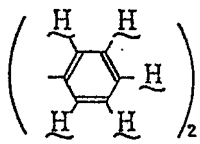 + —C 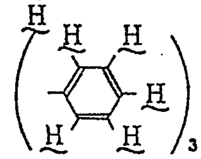

8.33 (2H, d, 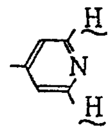 )

Example 1

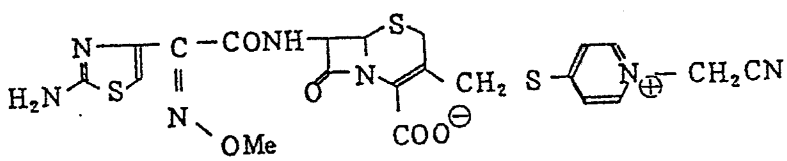

In 0.5 ml of dimethylsulfoxide was dissolved 150 mg of diphenylmethyl (Z)-7-[α-(2-tritylamino-4-thiazolyl)-α-(methoxyimino)acetamido]-3-(4-pyridinyl)thiomethyl-$\Delta^3$-

cephem-4-carboxylate. Then 0.5 ml of acetonitrile iodide was added to the solution. The mixture was stirred at room temperature for 7 hours. When 30 ml of ice water was poured into the reaction mixture, the system became thick and syrupy. The aqeuous phase was removed by decantation. The residue was again washed with 30 ml of ice water and 0.2 ml of anisole was added to the remaining thick syrup-like matter. Under ice cooling 15 ml of trifluoroacetic acid was added to the liquid. After stirring at room temperature for 40 minutes, 1.5 ml of water was added thereto under ice cooling. After the mixture was stirred for 1 hour at room temperature, the solution was concentrated under reduced pressure. To the residue, 100 ml of ether was added. The resulting solid was filtered, washed with ether and then dried. The powders were dissolved in water. The aqueous solution was subjected to chromatography using Diaion HP-20 (made by Mitsubishi Chemical Industry Co., Ltd.) and eluted firstly with water and then with water : methanol mixtures of regularly varying methanol proportion. Fractions containing the product were collected, concentrated and freeze dried to obtain 34 mg of (Z)-7-[α-(2-amino-4-thiazolyl)-α-(methoxyimino)acetamido]-3-(1-cyanomethyl-4-pyridinio)thiomethyl-$\Delta^3$-cephem-4-carboxylate.

NMR (DMSO - $d_6$)

δ : ppm 3.60 ( 2H, q, 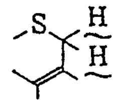 )

3.84 ( 3H, s, 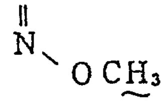 )

4.38 ( 2H, s, 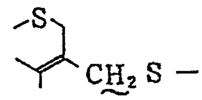 )

5.16 ( 1H, d, 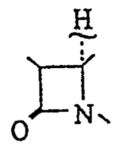 )

5.60 ~ 5.86 ( 3H, m, 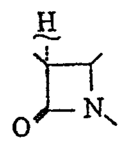 +

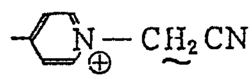 )

6.72 ( 1H, s, 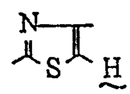 )

8.05 ( 2H, d, 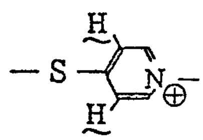 )

8.79 ( 2H, d, 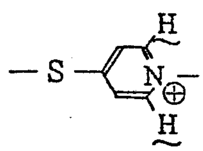 )

Example 2

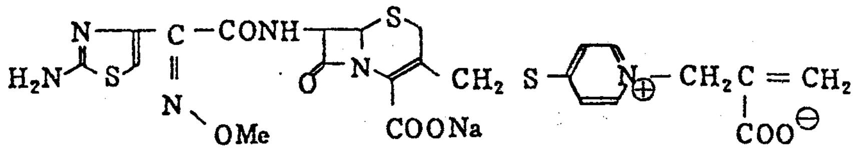

In 0.75 ml of dimethylsulfoxide was dissolved 200 mg of diphenylmethyl (Z)-7-[α-(2-tritylamino-4-thiazolyl)-α-(methoxyimino)acetamido]-3-(4-pyridinyl)thiomethyl- $\Delta^3$-cephem-4-carboxylate. Then 90 mg of t-butyl α-(bromomethyl)-acrylate was added to the solution. The mixture was stirred at room temperature for 2.5 hours. To the reaction mixture was added 0.2 ml of anisole . Under ice cooling 25 ml of trifluoroacetic acid was then added to the liquid. The mixture was stirred at room temperature for 1 hour. Under ice cooling, 4 ml of water was added to the reaction mixture and the mixture was stirred for 1 hour at room temperature. The reaction mixture was concentrated. To the residue, 100 ml of ether was added. The resulting powders were filtered off, washed with ether and then dried. The powders were dissolved in an aqueous solution of sodium hydrogen carbonate. The solution was subjected to chromatography using Diaion HP-20 and eluted firstly with water and then with water : methanol of regularly varying methanol proportion. Fractions containing the

product were collected, concentrated and freeze dried to obtain 33 mg of sodium (Z)-7-[α-(2-amino-4-thiazolyl)-α-(methoxyimino)acetamido]-3-[1-(2-carboxylate-2-propenyl)-4-pyridinio] thiomethyl-$\Delta^3$-cephem-4-carboxylate.

NMR (DMSO - $d_6$)

δ : ppm 3.80 (3H, s, 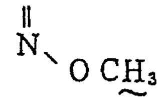 )

4.48 (2H, s, 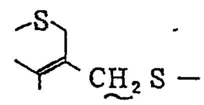 )

5.00 (1H, d, 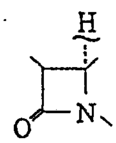 )

5.14 (2H, s, 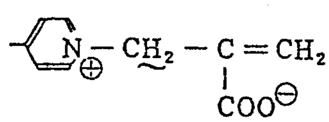 )

5.54 (1H, dd, 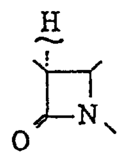 )

5.63, 6.04 (各々 1H, s,

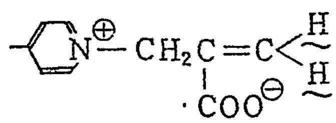 )

6.71 (1H, s, 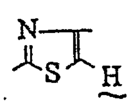 )

8.19 ( 2H, d, 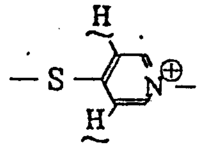 )

8.67 ( 2H, d, 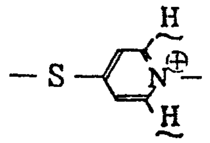 )

Example 3

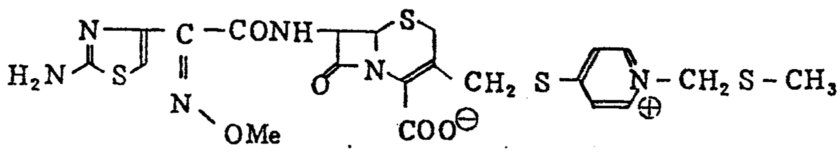

In 0.75 ml of dimethylsulfoxide was dissolved 200 mg of diphenylmethyl (Z)-7-[α-(2-tritylamino-4-thiazolyl)-α-(methoxyimino)acetamido]-3-(4-pyridinyl)thiomethyl-$\Delta^3$-cephem-4-carboxylate. Then 60 mg of chloromomethyl methylsulfide was added to the solution. The mixture was stirred at room temperature for 48 hours. To the reaction mixture was added 0.2 ml of anisole . Under ice cooling 25 ml of trifluoroacetic acid was then added to the liquid. The mixture was stirred at room temperature for 1 hour. Under ice cooling, 6 ml of water was added to the reaction mixture and the mixture was stirred for 1 hour at room temperature. The reaction mixture was concentrated. To the residue, 150 ml of ether was added. The resulting powders were filtered off

washed with ether and then dried. The powders were suspended in 200 ml of water. After the pH was adjusted to 7 ÷ 8 with an aqueous solution of sodium hydrogen carbonate, the system was subjected to chromatography using Diaion HP-20 and eluted firstly with water and then with water : methanol of regularly varying methanol proportion. Fractions containing the product were collected, concentrated and freeze dried to obtain 13 mg of (Z)-7-[α-(2-amino-4-thiazolyl)-α-(methoxyimino)acetamido]-3-[1-(methylthiomethyl)-4-pyridinio)thiomethyl- $\Delta^3$-cephem-4-carboxylate.

NMR (DMSO - $d_6$)

δ : ppm

2.12 (3H, s, $-CH_2SCH_3$ )

3.81 (3H, s, 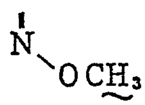 )

4.98 (1H, d, 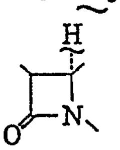 )

5.40 ~ 5.70 (3H, m, 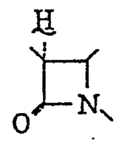 + $-CH_2SCH_3$ )

6.68 (1H, s, 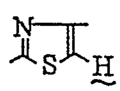 )

8.02 (2H, d, 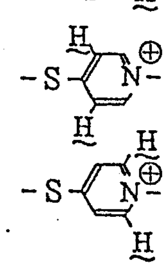 )

8.75 (2H, d, )

Example 4

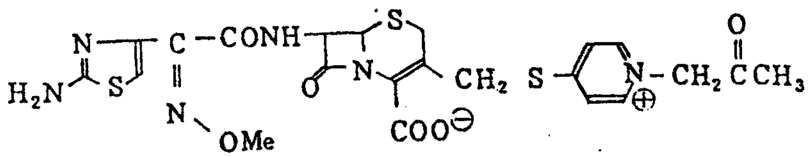

In 0.75 ml of dimethylsulfoxide was dissolved 300 mg of diphenylmethyl (Z)-7-[α-(2-tritylamino-4-thiazolyl)-α-(methoxyimino)acetamido]-3-(4-pyridinyl)thiomethyl-$\Delta^3$-cephem-4-carboxylate. Then 60 mg of chloroacetone was added to the solution. The mixture was stirred at room temperature for 41 hours. To the reaction mixture was added 0.2 ml of anisole. Under ice cooling 25 ml of trifluoroacetic acid was then added to the liquid. The mixture was stirred at room temperature for 1 hour. Under ice cooling, 6 ml of water was added to the reaction mixture and the mixture was stirred for 1 hour at room temperature. The reaction mixture was concentrated. To the residue, 200 ml of ether was added and the powders were filtered, washed with ether and then dried. The powders were dissolved in 150 ml of water. The solution was subjected to chromatography using Diaion HP-20 and eluted firstly with water and then with water : methanol of regularly varying methanol proportion. Fractions containing the product were collected, concentrated and freeze dried to obtain 71 mg of (Z)-7-[α-(2-amino-4-thiazolyl)-α-(methoxyimino)acetamido]-3-[1-acetonyl-4-pyridinio)thiomethyl-$\Delta^3$-cephem-4-carboxylate.

NMR ( DMSO$-d_6$ )

2.27 ( 3H, s, 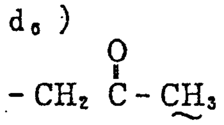 )

3.82 ( 3H, s, 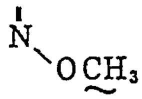 )

4.50 ( 2H, s, 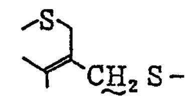 )

5.04 ( 1H, d, 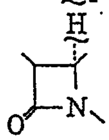 )

5.47 ~ 5.74 ( 3H, m, 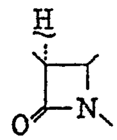 + 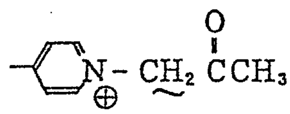

6.74 ( 1H, s, 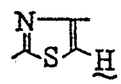 )

8.36 ( 2H, d, 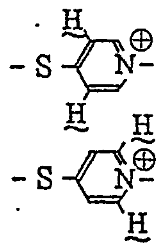 )

8.56 ( 2H, d, )

Example 5

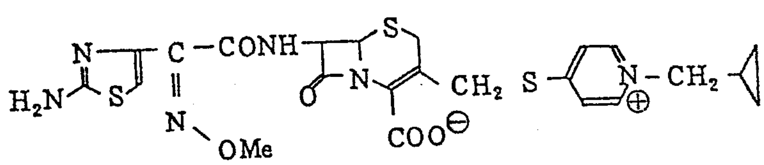

In 0.75 ml of dimethylsulfoxide was dissolved 300 mg of diphenylmethyl (Z)-7-[α-(2-tritylamino-4-thiazolyl)-α-(methoxyimino)acetamido]-3-(4-pyridinyl)thiomethyl- $\Delta^3$-cephem-4-carboxylate. Then 100 mg of bromomethylcyclopropane was added to the solution. The mixture was stirred at room temperature for 2 days. Then 100 mg of bromomethylcyclopropane was added thereto followd by stirring at room temperature for 2 days. Then was added 0.2 ml of anisole . Under ice cooling 25 ml of trifluoroacetic acid was then added to the liquid. The mixture was stirred at room temperature for 1 hour. Under ice cooling, 4 ml of water was added to the reaction mixture and the mixture was stirred for 1 hour at room temperature. The reaction mixture was concentrated. To the residue, 150 ml of ether was added and the powders were filtered, washed with ether and then dried. The powders were dissolved in 150 ml of water. The solution was subjected to chromatography using Diaion HP-20 and eluted firstly with water and then with water : methanol of regularly varying methanol proportion. Fractions containing the product were collected, concentrated and freeze dried to obtain 50 mg of (Z)-7-[α-(2-amino-4-thiazolyl)-α-(methoxyimino)acetamido]-3-(1-cyclopropylmethyl-4-pyridinio)thiomethyl- $\Delta^3$-cephem-4-carboxylate.

NMR ( DMSO-$d_6$ )

δ : ppm

0.20～0.74 ( 4H, m, 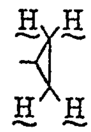 )

1.06～1.60 ( 1H, m, 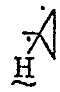 )

3.81 ( 3H, s, 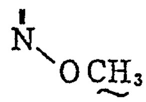 )

4.31 ( 2H, d, 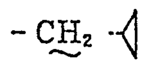 )

4.51 ( 2H, s, 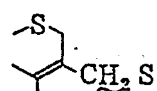 )

5.03 ( 1H, d, 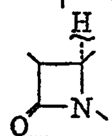 )

5.59 ( 1H, dd, 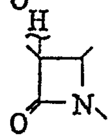 )

6.73 ( H, s, 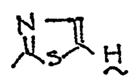 )

8.27 ( 2H, d, 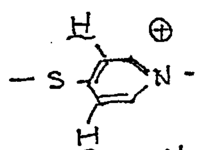 )

8.79 ( 2H, d, 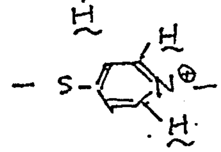 )

Reference Example 2:

(raw material for Example 6)

(a)

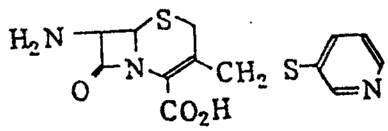

In 54 ml of water was suspended 3.6 g of 7-aminocephalosporanic acid and sodium hydrogen carbonate was added to the suspension little by little to dissolve. After 1.5 g of 3-mercaptopyridine was added to the solution, the resulting mixture was stirred at 55 to 60°C for 5 hours. After cooling, pH of the reaction mixture was adjusted to 3 with 6N hydrochloric acid. The precipitate was taken by filtration and dried to obtain 2.88 g of 7-amino-3-(3-pyridinyl)thiomethyl-$\Delta^3$-cephem-4-carboxylic acid.

i) NMR ($D_2O$ - DCl)

δ : ppm

3.83 (2H, s, 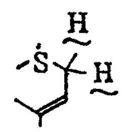 )

4.31 (2H, q, 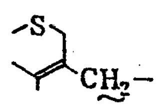 )

5.14, 5.32 (各々 1H, d, 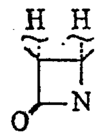 )

8.07 (1H, dd, 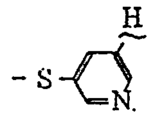 )

8.70 (1H, dd, 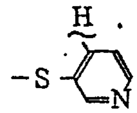 )

8.78 (1H, dd, 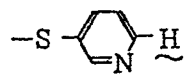 )

8.97 (1H, dd, 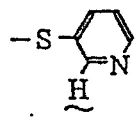 )

ii) FABMS $[M+H]^+$ 324

(b)

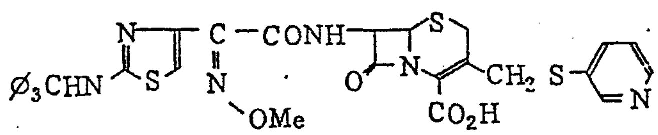

In 40 ml of dichloromethane was suspended 2.88 g of 7-amino-3-(3-pyridinyl)thiomethyl- $\Delta^3$-cephem-4-carboxylic acid and, 8.3 ml of N,O-bis(trimethylsilyl)trifluoroacetamide was added to the suspension. The mixture was stirred at room

temperature for 2 hours to form a homogeneous solution. The solution was cooled to -40°C and 2.81 ml of pyridine was added thereto (Liquid A).

On the other hand, 3.95 g of (Z)-α-(methoxyimino)-α-(2-tritylamino-4-thiazolyl)acetic acid was suspended in 40 ml of dichloromethane. After the suspension was cooled to -10°C, 1.86 g of phosphorus pentachloride was added to the suspension. The mixture was stirred for 15 minutes (Liquid B).

After dropwise adding Liquid B to Liquid A, the temperature was elevated to -15°C over 15 minutes and the mixture was stirred at this temperature for 10 minutes. To the solution were added 24 ml of 1N hydrochloric acid and 96 ml of water. After the mixture was stirred for 10 minutes under ice cooling, dichloromethane was removed from the reaction mixture by distillation under reduced pressure. The resulting powders were filtered off, washed with water and dried to obtain 5.9 g of crude (Z)-7-[α-(methoxyimino)-α-(2-tritylamino-4-thiazolyl)acetamido]-3-(3-pyridinyl)thiomethyl-$\Delta^3$-cephem-4-carboxylic acid.

(c)

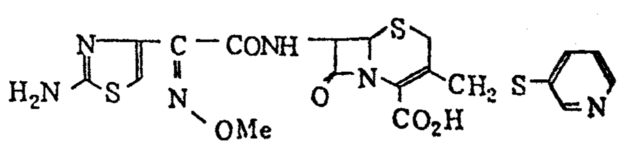

In 20 ml of trifluoroacetic acid cooled to 15°C was dissolved 748 mg of crude (Z)-7-[α-(methoxyimino)-α-(2-tritylamino-4-thiazolyl)acetamido]-3-(3-pyridinyl)thiomethyl-$\triangle^3$-cephem-4-carboxylic acid. To the solution was added 4 ml of water. The mixture was stirred at room temperature for 3 hours. The solvent was distilled off and 20 ml of ether was added to the residue to form powders. After filtering, the powders were dried. The powders were withdrawn and subjected to high speed liquid chromatography and eluted with a 20% acetonitrile-0.05 M potassium dihydrogen phosphate solution. The desired fractions were collected and concentrated. The residue was subjected to chromatography using Diaion HP-20 and eluted with a 50% methanol-water mixture. The eluted fractions were collected, concentrated and freeze dried to obtain 47 mg of (Z)-7-[α-(2-amino-4-thiazolyl)-α-(methoxyimino)acetamido]-3-(3-pyridinyl)thiomethyl-$\triangle^3$-cephem-4-carboxylic acid.

i) NMR (DMSO - $d_6$)

δ : ppm

3.77 (2H, q, 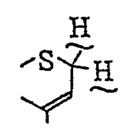 )

3.84 (3H, s, 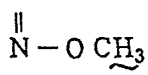 )

4.18 (2H, q, 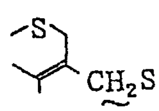 )

5.09 (1H, d, 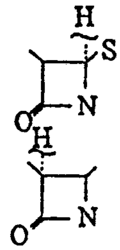 )

5.68 (1H, dd, )

6.76 (1H, s, 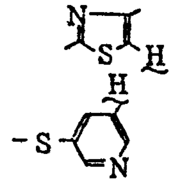 )

7.36 (1H, m, )

7.90 (1H, d, 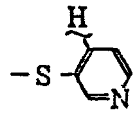 )

8.5 (2H, m, 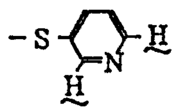 )

ii) FABMS $[M+H]^+$ 507

Example 6

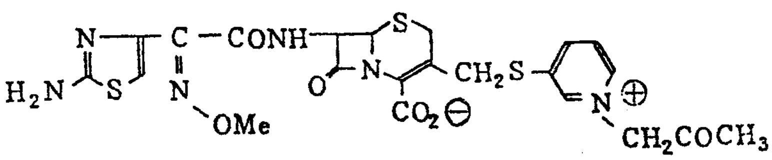

In 2 ml of dimethylsulfoxide was dissolved 1.5 g of crude (Z)-7-[α-methoxyimino-α-(2-tritylamino-4-thiazolyl)-acetamido]-3-(3-pyridinyl)thiomethyl- $\Delta^3$-cephem-4-carboxylic acid. Then 500 µl of chloroacetone was added to the solution. The mixture was reacted at room temperature for 72 hours. The reaction mixture was added to 7 ml of trifluoroacetic acid cooled to 10°C and 2 ml of water was further added thereto. The mixture was reacted at room

temperature for 1 hour. The reaction mixture was concentrated under reduced pressure. To the residue, ether was added to make powders. The powders were filtered and dried. The powders were treated with HP-20 subjected to high performance liquid chromatography and finally treated with HP-20 for desalting to obtain 73 mg of (Z)-7-[α-(2-amino-4-thiazolyl)-α-(methoxyimino)acetamido]-3-(1-acetonyl-4-pyridinio)thiomethyl- $\Delta^3$-cephem-4-carboxylate.

i) NMR (DMSO - $d_6$)

δ : ppm

2.30 (3H, s, $-CH_2\overset{O}{\overset{\|}{C}}CH_3$ )

3.82 (3H, s, 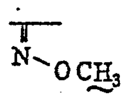 )

4.26 (2H, s, 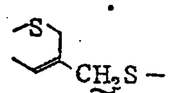 )

4.91 (1H, d, 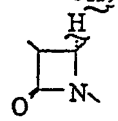 )

5.52 (1H, dd. 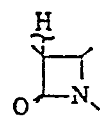 )

5.72 (2H, s, $-CH_2COCH_3$ )

6.73 (1H, s, 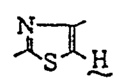 )

8.02 (1H, m, 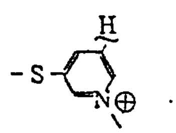 )

8.62 (2H, m, 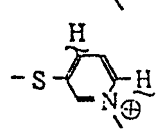 )

9.52 (1H, s, 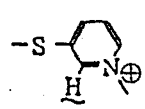 )

ii) FABMS $[M+H]^+$ 563

Example 7

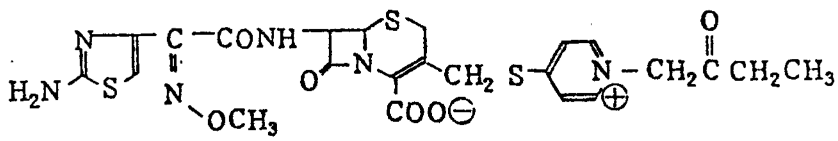

In 0.6 ml of dimethylsulfoxide was dissolved 300 mg of diphenylmethyl (Z)-7-[α-(2-tritylamino-4-thiazolyl)-α-(methoxyimino)acetamido]-3-(4-pyridinyl)thiomethyl-$\Delta^3$-cephem-4-carboxylate. Then 100 mg of 1-bromo-2-butanone was added to the solution. The mixture was stirred at room temperature for 30 minutes. To the reaction mixture was added 0.2 ml of anisole. Under ice cooling 25 ml of trifluoroacetic acid was then added to the liquid. The mixture was stirred at room temperature for 1 hour. Under ice cooling, 6 ml of water was added to the reaction mixture and the mixture was stirred for 1 hour at room temperature. The reaction mixture was concentrated. To the residue, 250 ml of ether was added. The powders were filtered off, washed with ether and then dried. The powders were dissolved in 100 ml of water. The solution was treated with Diaion HP-20 and eluted firstly with water and then with water : methanol of regularly varying methanol proportion. Fractions containing the product were collected, concentrated and freeze dried to obtain 105 mg of (Z)-7-[α-(2-amino-4-thiazolyl)-α-(methoxyimino)acetamido]-3-[1-(2-oxobutyl)-4-pyridinio)thiomethyl-$\Delta^3$-cephem-4-carboxylate.

NMR (DMSO-$d_6$)

δ : ppm

1.02 (3H, t, 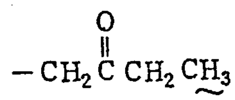 )

2.64 (2H, q, 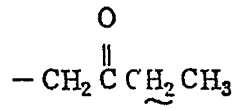 )

3.81 (3H, s, 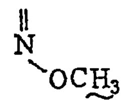 )

4.50 (2H, 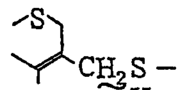 )

5.02 (1H, d, 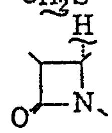 )

5.46 ~ 5.72 (3H, 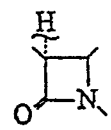 , 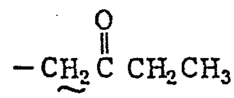 )

6.71 (1H, s, 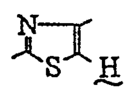 )

8.34 (2H, d, 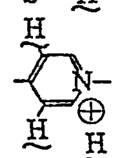 )

8.52 (2H, d, 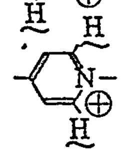 )

Example 8

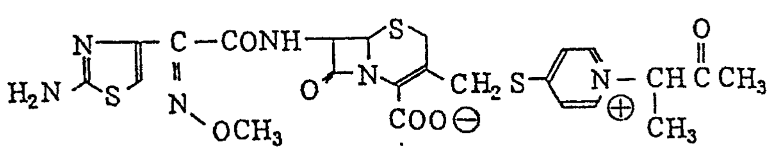

In dimethylsulfoxide was dissolved 506 mg of (Z)-7-[α-(2-amino-4-thiazolyl)-α-(methoxyimino)acetamido]-3-(4-pyridinyl)thiomethyl-$\Delta^3$-cephem-4-carboxylic acid. Then 215 mg of 3-chloro-2-butanone was added to the solution. Under ice cooling 330 mg of potassium iodide was added to the mixture. The resulting mixture was stirred at room temperature for 3 hours. Under ice cooling, 320 mg of 3-chloro-2-butanone and 500 mg of potassium iodide were added to the reaction mixture. After the mixture was stirred at room temperature for 1 hour, 535 mg of 3-chloro-2-butanone and 830 mg of potassium iodide were further added and stirred for 1.5 hours. To the reaction mixture was added ether to form thick syrupy matter. Ether was removed by decantation. After washing with ether several further times, ethyl acetate was added thereto to form powders. The powders were filtered and dissolved in 200 ml of water. Insoluble matters were removed by filtration. The system was treated with Diaion HP-20 and eluted firstly with water and then with water : methanol of regularly varying methanol proportion. Fractions containing the product were collected, concentrated and freeze dried to obtain 97 mg of (Z)-7-[α-(2-amino-4-thiazolyl)-α-(methoxyimino)acetamido]-3-[1-(1-methyl-2-oxopropyl)-4-pyridinio]thiomethyl-$\Delta^3$-cephem-4-carboxylate.

NMR (DMSO - $d_6$)

δ : ppm

1.8 7 ( 3 H, d, 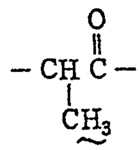 )

2.3 1 ( 3 H, s, 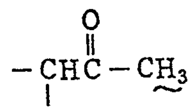 )

3.8 2 ( 3 H, s, 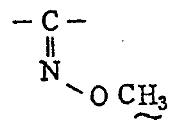 )

4.5 1 ( 2 H, m, 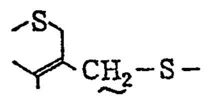 )

5.0 1 ( 1 H, d, 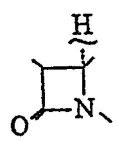 )

5.6 5 ( 1 H, m, 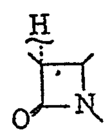 )

5.7 8 ( 1 H, m, 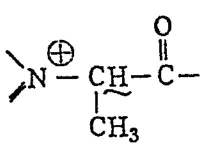 )

6.7 2 ( 1 H, s, 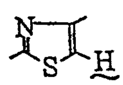 )

8.3 2 ( 2 H, d, 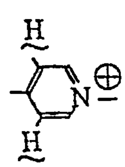 )

8.6 0 ( 2 H, d, 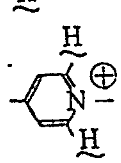 )

Reference Example 3

(raw material for Example 11)

(a)

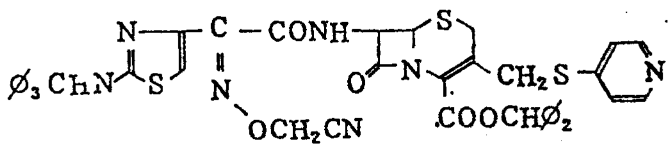

In 40 ml of dichloromethane was suspended 1.00 g of 7-amino-3-(4-pyridyl)thiomethyl- $\Delta^3$-cephem-4-carboxylic acid and, 3.97 g of N,O-bis(trimethylsilyl)trifluoroacetamide was added to the suspension. The mixture was stirred at room temperature for 5 hours to form a homogeneous solution. The solution was cooled to -50°C and 1.22 g of pyridine was added thereto (Liquid A).

On the other hand, 1.45 g of (Z)-α-(2-tritylamino-4-thiazolyl)-α-(cyanomethoxyimino)acetic acid was suspended in 10 ml of dichloromethane. Under ice cooling, 0.64 g of phosphorus pentachloride was added to the suspension. The mixture was stirred for 20 minutes (Liquid B).

After dropwise adding Liquid B to Liquid A, the temperature was elevated to -15°C over 15 minutes and the mixture was stirred at this temperature for 10 minutes. To the solution were added 20 ml of 1N hydrochloric acid, 20 ml of water and 10 ml of tetrahydrofuran. After the mixture was stirred for 10 minutes under ice cooling, dichloromethane and

tetrahydrofuran were removed from the reaction mixture by distillation under reduced pressure. To the residue 200 ml of water was added. The mixture was ground into powders, filtered, washed with water and dried to obtain 2.30 g of crude (Z)-7-[α-(2-tritylamino-4-thiazolyl)-α-(cyanomethoxyimino)acetamido]-3-(4-pyridyl)thiomethyl- $\Delta^3$-cephem-4-carboxylic acid.

(b) In 50 ml of dichloromethane was dissolved 2.30 g of crude (Z)-7-[α-(2-tritylamino-4-thiazolyl)-α-(cyanomethoxyimino)acetamido]-3-(4-pyridyl)thiomethyl- $\Delta^3$-cephem-4-carboxylic acid. Under ice cooling 0.58 g of diphenyldiazomethane was added to the solution. After stirring at room temperature for 1 hour, the reaction mixture was concentrated. The residue was subjected to silica gel chromatography and eluted firstly with chloroform and then with chloroform : ethyl acetate of regularly changing proportions. From fractions containing the product, 0.7 g of diphenylmethyl (Z)-7-[α-(2-tritylamino-4-thiazolyl)-α-(cyanomethoxyimino)acetamido]-3-(4-pyridyl)thiomethyl- $\Delta^3$-cephem-4-carboxylate was obtained.

NMR ( DMSO − $d_6$ )

δ : ppm 3.61 ( 2H, q, 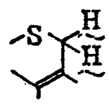 )

4.00 − 4.22 ( 2H, 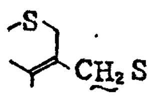 )

4.96 ( 2H, s, 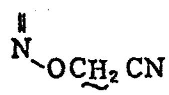 )

5.19 ( 1H, d, 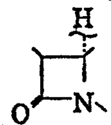 )

5.73 ( 1H, dd, 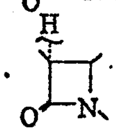 )

6.85 ( 1H, s, 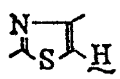 )

6.94 ( 1H, s, − COOCH $Ø_2$ )

7.08 ~ 7.58 ( 27H, m,

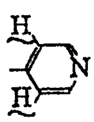 + 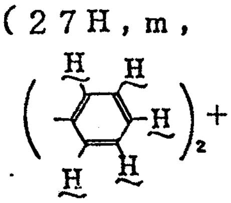 + 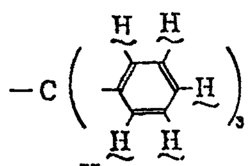 )

8.20 ~ 8.40 ( 2H, m, 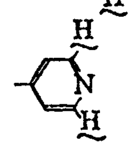 )

Reference Example 4

(raw material for Example 12)

(a)

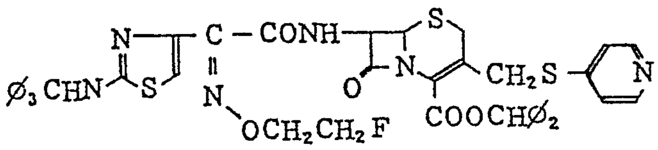

In 30 ml of dichloromethane was suspended 1.50 g of 7-amino-3-(4-pyridyl)thiomethyl- $\Delta^3$-cephem-4-carboxylic acid and, 7.16 g of N,O-bis(trimethylsilyl)trifluoroacetamide was added to the suspension. The mixture was stirred at room temperature for 5 hours to form a homogeneous solution. The solution was cooled to -50°C and 2.02 g of pyridine was added thereto (Liquid A).

On the other hand, 2.21 g of (Z)-α-(2-tritylamino-4-thiazolyl)-α-(2-fluoroethoxyimino)acetic acid was suspended in 20 ml of dichloromethane. Under ice cooling, 1.06 g of phosphorus pentachloride was added to the suspension. The mixture was stirred for 30 minutes (Liquid B).

After dropwise adding Liquid B to Liquid A, the temperature was elevated to -15°C over 15 minutes and the mixture was stirred at this temperature for 10 minutes. To the solution were added 30 ml of 1N hydrochloric acid, 15 ml of water and 15 ml of tetrahydrofuran. After the mixture was stirred for 10 minutes under ice cooling, dichloromethane and tetrahydrofuran were removed from the reaction mixture by

distillation under reduced pressure. To the residue 250 ml of water was added. The mixture was ground into powders, filtered, washed with water and dried to obtain 3.70 g of crude (Z)-7-[α-(2-tritylamino-4-thiazolyl)-α-(2-fluoroethoxyimino)acetamido]-3-(4-pyridyl)thiomethyl- $\Delta^3$-cephem-4-carboxylic acid.

(b) In 70 ml of dichloromethane was dissolved 3.70 g of crude (Z)-7-[α-(2-tritylamino-4-thiazolyl)-α-(2-fluoroethoxyimino)acetamido]-3-(4-pyridyl)thiomethyl- $\Delta^3$-cephem-4-carboxylic acid. Under ice cooling 0.92 g of diphenyldiazomethane was added to the solution. After stirring at room temperature for 1 hour, the reaction mixture was concentrated. The residue was subjected to silica gel chromatography and eluted firstly with chloroform and then with chloroform : ethyl acetate of regularly changing proportions. From fractions containing the product, 1.1 g of diphenylmethyl (Z)-7-[α-(2-tritylamino-4-thiazolyl)-α-(2-fluoroethoxyimino)acetamido]-3-(4-pyridyl)thiomethyl- $\Delta^3$-cephem-4-carboxylate was obtained.

NMR ( DMSO − $d_6$ )

δ : ppm  3.62 ( 2H,  q,  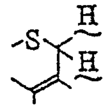 )

4.00∼4.20 ( 3H,  m,  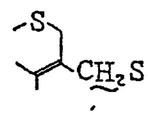 + 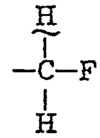 )

4.24∼4.52 ( 2H,  m,  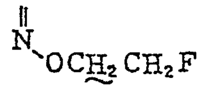 )

4.76∼4.94 ( 1H,  m,  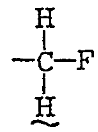 )

5.21 ( 1H,  d,  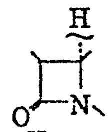 )

5.66 ( 1H,  dd,  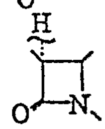 )

6.76 ( 1H,  s,  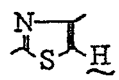 )

6.96 ( 1H,  s,  −COOCH∅$_2$ )

7.08∼7.60 ( 27H,  m,

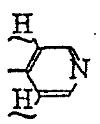 + 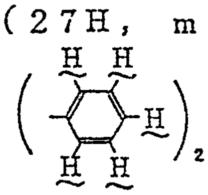 + −C 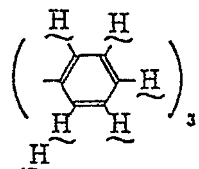 )

8.24∼9.40 ( 2H,  m,  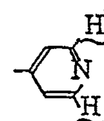 )

Example 9

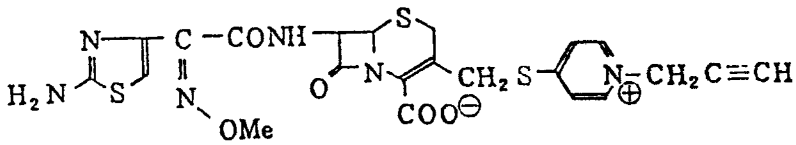

In 0.75 ml of dimethylsulfoxide was dissolved 300 mg of diphenylmethyl (Z)-7-[α-(2-tritylamino-4-thiazolyl)-α-(methoxyimino)acetamido]-3-(4-pyridyl)thiomethyl- $\Delta^3$-cephem-4-carboxylate. Then 90 mg of propargyl bromide was added to the solution. The mixture was stirred at room temperature for 24 hours. To the reaction mixture was added 0.2 ml of anisole. Under ice cooling 30 ml of trifluoroacetic acid was added to the liquid. After stirring at room temperature for 30 minutes, 6 ml of water was added thereto under ice cooling. After the mixture was stirred for 1 hour at room temperature, the reaction mixture was concentrated. To the residue, 150 ml of ether was added to form powders. The powders were filtered, washed with ether and then dried. The powders, 230 mg, were dissolved in 150 ml of water. The solution was treated with Diaion HP-20 (made by Mitsubishi Chemical Industry Co., Ltd.) and eluted firstly with water and then with water : methanol of regularly changing methanol proportion. Fractions containing the product were collected, concentrated and freeze dried to obtain 86 mg of (Z)-7-[α-(2-amino-4-thiazolyl)-α-(methoxyimino)acetamido]-3-[1-propargyl-4-pyridinio]thiomethyl- $\Delta^3$-cephem-4-carboxylate.

NMR ( DMSO-$d_6$ )

δ : ppm

3.84 ( 3H, s, 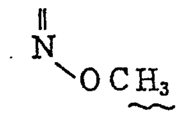 )

3.92 ( 1H, s, $-CH_2C\equiv CH$ )

4.52 ( 2H, s, 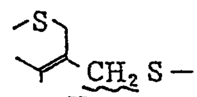 )

5.02 ( 1H, d, 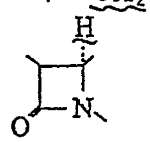 )

5.46 ( 2H, s, $-CH_2C\equiv CH$ )

5.98 ( 1H, dd, 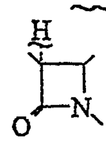 )

6.75 ( 1H, s, 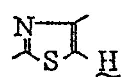 )

8.36 ( 2H, d, 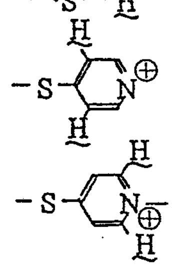 )

8.56 ( 2H, d, )

Example 10

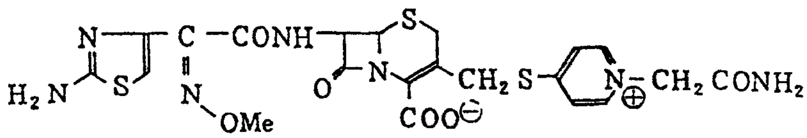

In 0.75 ml of dimethylsulfoxide was dissolved 300 mg of diphenylmethyl (Z)-7-[α-(2-tritylamino-4-thiazolyl)-α-(methoxyimino)acetamido]-3-(4-pyridyl)thiomethyl- $\Delta^3$-cephem-4-carboxylate. Then 185 mg of iodoacetamide was added to the solution. The mixture was stirred at room temperature for 20 hours. To the reaction mixture was added 0.3 ml of anisole . Under ice cooling 30 ml of trifluoroacetic acid was then added to the liquid. The mixture was stirred at room temperature for 30 hours. Under ice cooling, 6 ml of water was added to the reaction mixture and the mixture was stirred for 1 hour at room temperature. The reaction mixture was concentrated. To the residue, 150 ml of ether was added, and the powders were filtered off, washed with ether and then dried. The powders were dissolved in 150 ml of water. The solution was treated with Diaion HP-20 and eluted firstly with water and then with water : methanol of regularly changing methanol proportion. Fractions containing the product were collected, concentrated and freeze dried to obtain 77 mg of

(Z)-7-[α-(2-amino-4-thiazolyl)-α-(methoxyimino)acetamido]-3-[1-carbamoylmethyl-4-pyridinio]thiomethyl-$\Delta^3$-cephem-4-carboxylate.

NMR (DMSO - $d_6$)

δ : ppm

3.82 ( 3H, s, 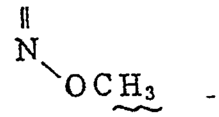 )

4.51 ( 2H, q, 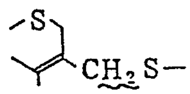 )

5.04 ( 1H, d, 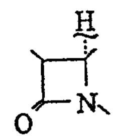 )

5.28 ( 2H, s, $-CH_2CONH_2$ )

5.58 ( 1H, dd, 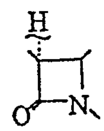 )

6.73 ( 1H, s, 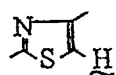 )

8.34 ( 2H. d, 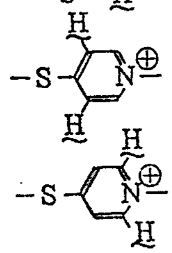 )

8.65 ( 2H, d, )

Example 11

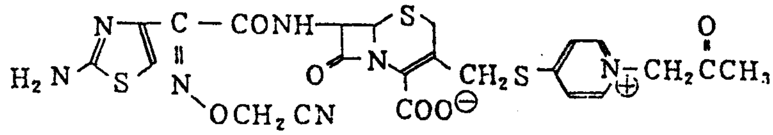

In 0.6 ml of dimethylsulfoxide was dissolved 300 mg of diphenylmethyl (Z)-7-[α-(2-tritylamino-4-thiazolyl)-α-(cyanomethoxyimino)acetamido]-3-(4-pyridyl)thiomethyl- $\Delta^3$-cephem-4-carboxylate. Then 90 mg of bromoacetone was added to the solution. The mixture was stirred at room temperature for 1.5 hours. To the reaction mixture was added 0.2 ml of anisole . Under ice cooling 25 ml of trifluoroacetic acid was then added to the liquid. The mixture was stirred at room temperature for 1 hour. Under ice cooling, 6 ml of water was added to the reaction mixture and the mixture was stirred for 1 hour at room temperature. After the reaction mixture was concentrated, 200 ml of ether was added to the residue to form powders. The powders were filtered, washed with ether and the dried. The powders were dissolved in 150 ml of water. The solution was treated with Diaion HP-20 and eluted firstly with water and then with water : methanol of regularly changing methanol proportion. Fractions containing the product were collected, concentrated and freeze dried to obtain 87 mg of (Z)-7-[α-(2-amino-4-thiazolyl)-α-(cyanomethoxyimino)acetamido]-3-[1-acetonyl-4-pyridinio)thiomethyl- $\Delta^3$-cephem-4-carboxylate.

NMR (DMSO-$d_6$)

δ : ppm

2.26 (3H, s, 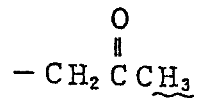 )

4.51 (2H, ABq 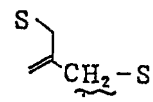 )

5.00 (2H, s, 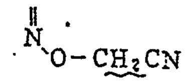 )

5.03 (1H, d, 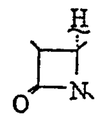 )

5.44～5.68 (3H, 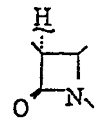 , 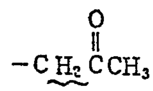 )

6.84 (1H, s, 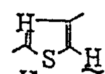 )

8.37 (2H, d, 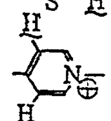 )

8.52 (2H, d, 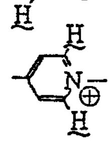 )

Example 12

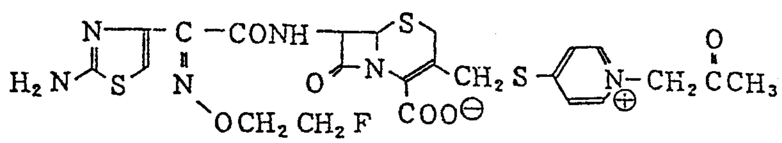

In 6 ml of dimethylsulfoxide was dissolved 300 mg of diphenylmethyl (Z)-7-[α-(2-tritylamino-4-thiazolyl)-α-(2-fluoroethoxyimino)acetamido]-3-(4-pyridyl)thiomethyl-$\Delta^3$-cephem-4-carboxylate. Then 87 mg of bromoacetone was added to the solution. The mixture was stirred at room temperature for 20 minutes. To the reaction mixture was added 0.2 ml of anisole . Under ice cooling 25 ml of trifluoroacetic acid was then added to the liquid. The mixture was stirred at room temperature for 1 hour. Under ice cooling, 6 ml of water was added to the reaction mixture and the mixture was

stirred for 1 hour at room temperature. The reaction mixture was concentrated. To the residue, 200 ml of ether was added and the powders were filtered off, washed with ether and then dried. The powders were dissolved in 150 ml of water. The solution was treated with Diaion HP-20 and eluted firstly with water and then with water : methanol of regularly changing methanol proportion. Fractions containing the product were collected, concentrated and freeze dried to obtain 67 mg of (Z)-7-[α-(2-amino-4-thiazolyl)-α-(2-fluoroethoxyimino)acetamido]-3-[1-acetonyl-4-pyridinio]thiomethyl- $\Delta^3$-cephem-4-carboxylate.

NMR ( DMSO-$d_6$ )

δ : ppm

2.28 ( 3 H, s, $-CH_2\overset{\overset{O}{\|}}{C}C\underset{\sim}{H_3}$ )

4.14 ( 1 H, m, $-CH_2\underset{H}{\overset{F}{C}}-\underset{\sim}{H}$ )

4.28 ~ 4.62 ( 4 H, m, $-O-\underset{\sim}{CH_2}-CH_2F$, 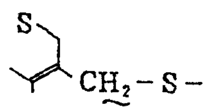 )

4.89 ( 1 H, m, $-CH_2-\underset{H}{\overset{F}{C}}-\underset{\sim}{H}$ )

5.03 ( 1 H, d, 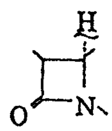 )

5.48 ~ 5.72 ( 3 H, $-\underset{\sim}{CH_2}\overset{\overset{O}{\|}}{C}CH_3$ , 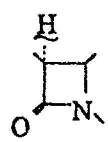 )

6.75 ( 1 H, s, 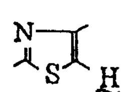 )

8.36 ( 2 H, d, 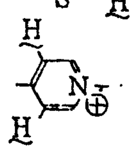 )

8.51 ( 2 H d, 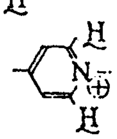 )

Example 13

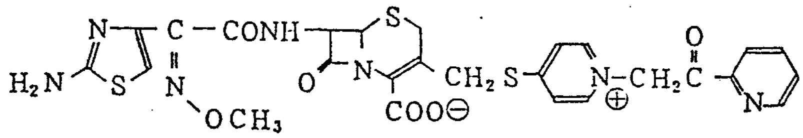

In 0.6 ml of dimethylsulfoxide was dissolved 300 mg of diphenylmethyl (Z)-7-[α-(2-tritylamino-4-thiazolyl)-α-(methoxyimino)acetamido]-3-(4-pyridyl)thiomethyl- $\Delta^3$-cephem-4-carboxylate. Then 131 mg of 2-bromoacetylpyridine was added to the solution. The mixture was stirred at room temperature for 1 hour. To the reaction mixture was added 0.2 ml of anisole . Under ice cooling 25 ml of trifluoroacetic acid was then added to the liquid. The mixture was stirred at room temperature for 1 hour. Under ice cooling, 6 ml of water was added to the reaction mixture and the mixture was stirred for 1 hour at room temperature. The reaction mixture was concentrated. To the residue, 300 ml of ether was added to make powders. The powders were filtered, washed with ether and then dried. The powders were dissolved in 150 ml of water. The solution was treated with Diaion HP-20 and eluted firstly with water and then with water : methanol, of regularly changing methanol proportion. Fractions containing the product were collected, concentrated and freeze dried to obtain 113 mg of (Z)-7-[α-(2-amino-4-thiazolyl)-α-(methoxyimino)acetamido]-3-(1-(2-pyridyl-2-oxoethyl)-4-pyridinio)-thiomethyl- $\Delta^3$-cephem-4-carboxylate.

NMR ( DMSO-$d_6$ )

δ : ppm

3.82 ( 3H, s, 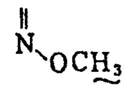 )

4.53 ( 2H, q, 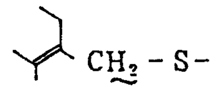 )

5.03 ( 1H, d, 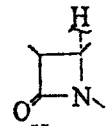 )

5.57 ( 1H, dd, 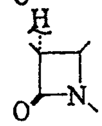 )

6.30 ( 2H, s, 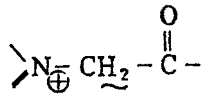 )

6.72 ( 1H, s, 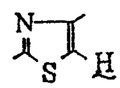 )

7.68 - 7.90 ( 1H, m, 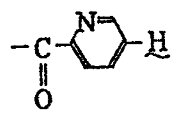 )

7.96 - 8.16 ( 2H, m, 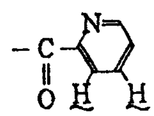 )

8.41 ( 2H, d, 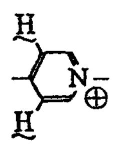 )

8.58 ( 2H, d, 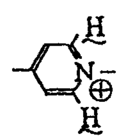 )

8.84 ( 1H, d, 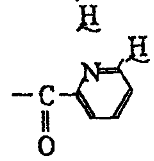 )

Example 14

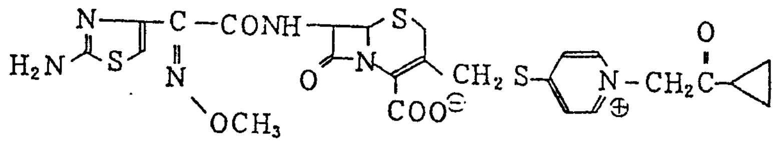

In 0.6 ml of dimethylsulfoxide was dissolved 300 mg of diphenylmethyl (Z)-7-[α-(2-tritylamino-4-thiazolyl)-α-(methoxyimino)acetamido]-3-(4-pyridyl)thiomethyl- $\Delta^3$-cephem-4-carboxylate. Then 107 mg of bromomethyl cyclopropyl ketone was added to the solution. The mixture was stirred at room temperature for 30 minutes. To the reaction mixture was added 0.2 ml of anisole. Under ice cooling 25 ml of trifluoroacetic acid was then added to the liquid. The mixture was stirred at room temperature for 1 hour. Under ice cooling, 6 ml of water was added to the reaction mixture and the mixture was stirred for 1 hour at room temperature. The reaction mixture was concentrated. To the residue, 300 ml of ether was added to make powders. The powders were filtered, washed with ether and then dried. The powders were dissolved in 150 ml of water. The solution was treated with Diaion HP-20 and eluted firstly with water and then with water : methanol of regularly changing methanol proportion. Fractions containing the product were collected, concentrated and freeze dried to obtain 108 mg of (Z)-7-[α-(2-amino-4-thiazolyl)-α-(methoxyimino)acetamido]-3-(1-(2-cyclopropyl-2-oxo)ethyl-4-pyridinio)thiomethyl- $\Delta^3$-cephem-4-carboxylate.

NMR ( DMSO - $d_6$ )

δ : ppm

0.80～1.28 ( 4H, m, 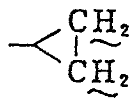 )

2.04～2.40 ( 1H, m, - CH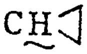 )

3.80 ( 3H, s, $=N-OCH_3$ )

4.50 ( 2H, q, 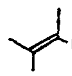$CH_2$ - S - )

5.00 ( 1H, d, 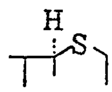 )

5.54 ( 1H, dd, 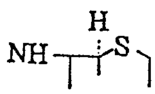 )

6.70 ( 1H, s, 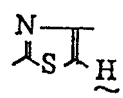 )

8.32 ( 2H, d, 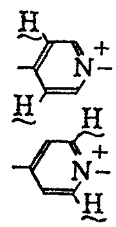 )

8.52 ( 2H, d, )

Example 15

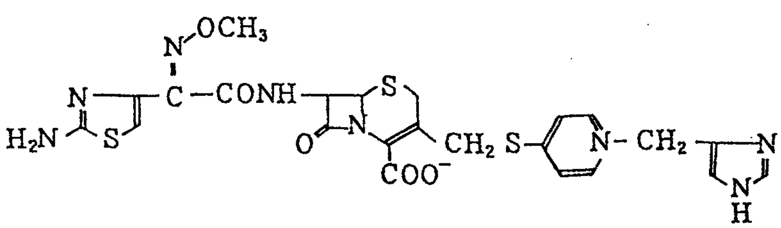

In 1 ml of dimethylsulfoxide was dissolved 200 mg of (Z)-7-[α-(2-amino-4-thiazolyl)-α-(methoxyimino)acetamido]-3-(4-pyridinyl)thiomethyl-$\Delta^3$-cephem-4-carboxylic acid. While keeping the inner temperature at -5 to -10°C, 196 mg of 4-chloromethylimidazole hydrochloride was added to the solution, in 4 separate portions. After the mixture was stirred at the same temperature for 15 minutes, the reaction mixture was dispersed in a 5% aqueous sodium hydrogen carbonate solution. The resulting solution was subjected to column chromatography using Diaion HP-20 and eluted firstly with water and then with water : methanol of regularly changing methanol proportion. Fractions containing the product were collected, concentrated and freeze dried to obtain 78.6 mg of (Z)-7-[α-(2-amino-4-thiazolyl)-α-(methoxyimino)acetamido]-3-(1-(4-imidazolyl)methyl-4-pyridinio)thiomethyl-$\Delta^3$-cephem-4-carboxylate.

0190900

NMR ( DMSO-$d^6$ )

$\delta$ = ppm

3.42 (2H, q, 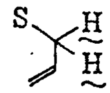 )

3.80 (3H, s, 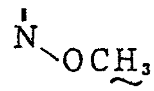 )

4.46 (2H, m, 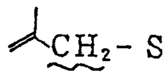 )

4.98 (1H, d, 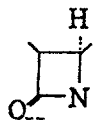 )

5.44～5.64 (3H, 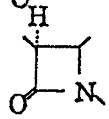 , 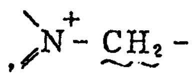 )

6.70 (1H, s, 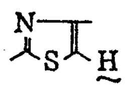 )

7.36, 7.66 (それぞれ 1H, 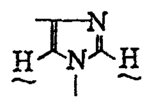 )

8.26 (2H, d, 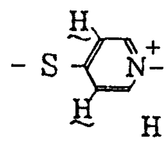 )

8.72 (2H, d, 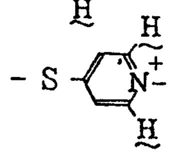 )

Example 16

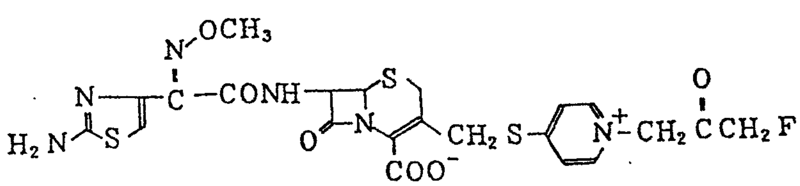

In 2 ml of dimethylsulfoxide was dissolved 400 mg of (Z)-7-[α-(2-amino-4-thiazolyl)-α-(methoxyimino)acetamido]-3-(4-pyridyl)thiomethyl- $\Delta^3$-cephem-4-carboxylic acid. To the solution was added 271 mg of 1-chloro-3-fluoroacetone. After stirring at room temperature for 8 hours, a further 200 mg of 1-chloro-3-fluoroacetone was added followed by stirring at room temperature for 20 hours. A further 200 mg of 1-chloro-3-fluoroacetone was added thereto. After stirring at room temperature for 6 hours, the reaction mixture was dispersed in 50 ml of ether. The formed precipitate was collected by decantation and washed with ether by decantation. The thus obtained solid was dissolved in water. The resulting solution was subjected to column chromatography using Diaion HP-20 and eluted firstly with water and then with water : methanol of regularly changing methanol proportion. Fractions containing the product were collected, concentrated and freeze dried to obtain 90 mg of (Z)-7-[α-(2-amino-4-thiazolyl)-α-(methoxyimino)acetamido]-3-(1-(3-fluoro-2-oxopropyl)-4-pyridinio)thiomethyl- $\Delta^3$-cephem-4-carboxylate. From the analyse of NMR and FABS, it is considered that the product would partly form the hydrate

( 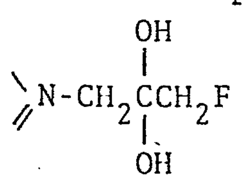 ).

NMR(DMSO-$d^6$) δ: ppm

3.52(2H, q)

3.80(3H, s)

4.0～4.8(broad,)

5.08(1H, dd)

5.5 - 5.8(broad)

6.70(1H, s)

8.0 - 8.64(m)

FABMS $(M+H)^+$ 581; $(M+H_2O+H)^+$ 599

Example 17

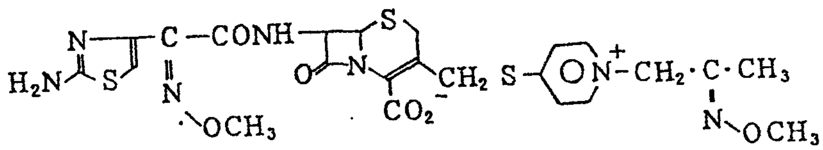

In 3.75 ml of N,N-dimethylformamide was dissolved 750 mg of (Z)-7-[α-(2-amino-4-thiazolyl)-α-(methoxyimino)acetamido]-3-(4-pyridyl)thiomethyl-$\Delta^3$-cephem-4-carboxylic acid—$H_2SO_4$ salt. Then 84.3 mg of potassium carbonate powder were added to the solution. After the mixture was stirred at room temperature for 30 minutes, the system was ice-cooled. After adding 934 µl of N,O-bistrimethylsilyl acetamide thereto, the mixture was stirred at 0 to 5°C for 1.5 hours. Then, 608 mg of 1-bromo-2-methoxyiminopropane was added to the reaction mixture. The mixture was reacted at room temperature for 4 days.

The reaction solution was dispersed in 20 ml of isopropanol. The precipitated solid was taken by filtration, washed with isopropanol-ethyl acetate (1:1) and then ethyl acetate and dried. The obtained powders were subjected to column chromatography using HP-20 and eluted with water :

methanol of regularly changing methanol proportion. Fractions containing the product were collected, concentrated and freeze dried to obtain 210 mg of (Z)-7-[α-(2-amino-4-thiazolyl)-α-(methoxyimino)acetamido]-3-(1-(2-methoxyiminopropyl)-4-pyridinio)thiomethyl- $\Delta^3$-cephem-4-carboxylate.

i) NMR ($d^6$-DMSO) δ : ppm

1.85 (3H, s, 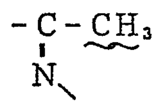 )

3.70 (3H, s, 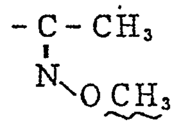 )

3.82 (3H, s, 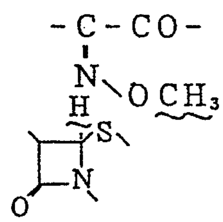 )

5.00 (1H, d, )

5.25 (2H, s, 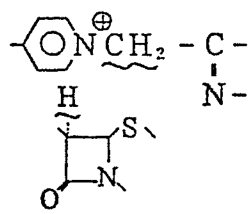 )

5.76 (1H, dd, )

6.72 (1H, s, 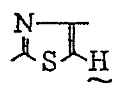 )

8.24 (2H, d, 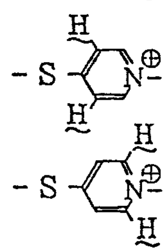 )

8.63 (2H, d, )

ii) IR (KBr) $cm^{-1}$

1760, 1680, 1615, 1525

iii) FABMS $[M+H]^+$

592

Example 18

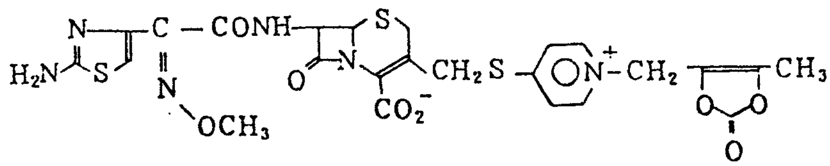

In 3.75 ml of N,N-dimethylformamide was dissolved 750 mg of (Z)-7-[α-(2-amino-4-thiazolyl)-α-(methoxyimino)acetamido]-3-(4-pyridyl)thiomethyl-$\Delta^3$-cephem-4-carboxylate sulfate. Then 84.3 mg of potassium carbonate powder were added to the solution. After the mixture was stirred at room temperature for 30 minutes, the solution was ice-cooled. After adding 934 μl of N,O-bistrimethylsilyl acetamide thereto, the mixture was stirred at 0 to 5°C for 1.5 hours. Then, 707 mg of 4-bromomethyl-5-methyl-1,3-dioxolen-2-one was added to the reaction mixture. The mixture was reacted at 5°C for 40 hours.

The reaction solution was dispersed in 2 ml of isopropanol. The precipitated solid was taken by filtration, washed with isopropanol-ethyl acetate (1:1) and then ethyl acetate and dried. The obtained powders were subjected to column chromatography using HP-20 and eluted with water : methanol of regularly changing methanol proportion. Fractions containing the product were collected, concentrated and freeze dried to obtain 130 mg of (Z)-7-[α-(2-amino-4-thiazolyl)-α-(methoxyimino)acetamido]-3-[1-(5-methyl-2-oxo-1,3-dioxolen-4-yl)methyl-4-pyridinio]thiomethyl-$\Delta^3$-cephem-4-carboxylate.

i) NMR (DMSO - $d_6$)

i) NMR ( $d^6$ - DMSO ) $\delta$ : ppm

2.26 ( 3H, s, 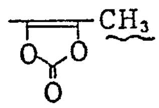 )

3.80 ( 3H, s, 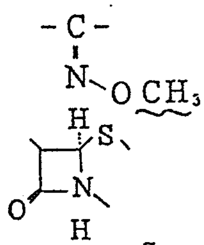 )

5.03 ( 1H, d, )

5.58 ( 1H, dd, 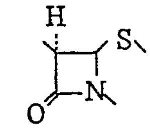 )

5.68 ( 2H, s, 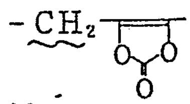 )

6.70 ( 1H, s, 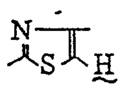 )

8.23 ( 2H, d, 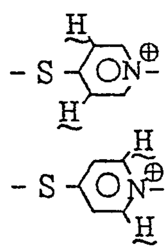 )

8.72 ( 2H, d, )

ii) IR ( KBr ) $cm^{-1}$

1810, 1760, 1660, 1620

iii) FABMS $[M+H]^+$

619

Example 19

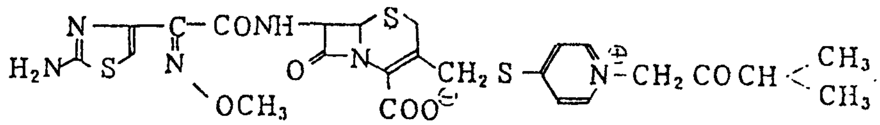

In 37.5 ml of N,N-dimethylformamide was dissolved 7.5 g of (Z)-7-[α-(2-amino-4-thiazolyl)-α-(methoxyimino)acetamido]-3-(4-pyridyl)thiomethyl- $\Delta^3$-cephem-4-carboxylic acid-$H_2SO_4$ salt. Then 843 mg of potassium carbonate powder were added to the solution. After the mixture was stirred at room temperature for 30 minutes, the solution was ice-cooled. While ice-cooling this solution, 4 ml of 1-bromo-3-methyl-2-butanone was added thereto. The mixture was stirred for 4 hours under ice cooling and 1 hour at room temperature. The reaction solution was poured into 200 ml of isopropanol. The precipitated solid was taken by filtration. The solid was dissolved in 400 ml of water and subjected to column chromatography using HP-20. Fractions containing the product were collected, concentrated and then powdered with isopropanol. The powders were taken by filtration and dried to obtain 3.8 g of (Z)-7-[α-(2-amino-4-thiazolyl)-α-(methoxyimino)acetamido]-3-[1-(3-methyl-2-oxo-butyl)-4-pyridinio]-thiomethyl- $\Delta^3$-cephem-4-carboxylate.

NMR (DMSO-$d_6$)

δ : ppm 1.12 (6H, d, $-CH<^{CH_3}_{CH_3}$ )

2.86 (1H, m, $-CH_2CH<^{CH_3}_{CH_3}$ )

3.50 (2H, q, $C_2-CH_2$ )

3.82 (3H, s, $-OCH_3$ )

4.50 (2H, q, $C_3-CH_2$ )

5.04 (1H, d, $C_6-H$ )

5.58 (1H, dd, $C_7-H$ )

5.84 (2H, b, $-CH_2CO-$ )

6.52 (1H, s, 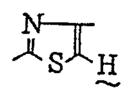 )

8.31 (2H, d, 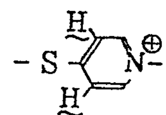 )

8.63 (2H, d, 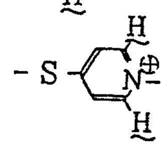 )

9.53 (1H, d, $-CONH-$ )

Example 20

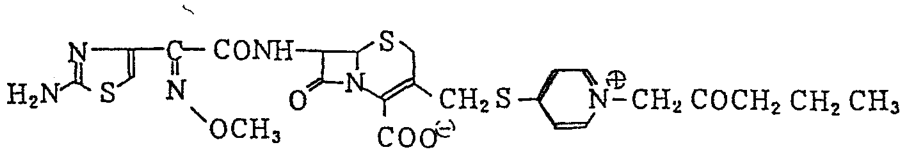

(Z)-7-[α-(2-Amino-4-thiazolyl)-α-(methoxyimino)acetamido]-3-[(1-(2-oxopentyl)-4-pyridinio]thiomethyl-$\Delta^3$-cephem-4-carboxylate was obtained in an amount of 3.6 g in a manner similar to the example described using 1-bromo-2-pentanone instead of 1-bromo-3-methyl-2-butanone.

NMR ( DMSO - $d_6$ )

δ : ppm 0.88 ( 3H, t, $-CH_2CH_3$ )

1.56 ( 2H, m, $-CH_2CH_2CH_3$ )

2.60 ( 2H, t, $-CH_2CH_2CH_3$ )

3.48 ( 2H, q, $C_2-CH_2-$ )

3.76 ( 3H, s, $-OCH_3$ )

4.48 ( 2H, q, $C_3-CH_2-$ )

5.02 ( 1H, d, $C_6-H$ )

5.54 ( 1H, dd, $C_7-H$ )

5.66 ( 2H, b, $-CH_2COCH_2CH_3$ )

6.50 ( 1H, s, 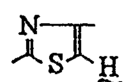 )

8.32 ( 2H, d, -S 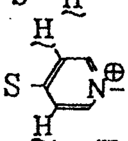 )

8.58 ( 2H, d, -S 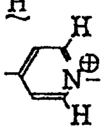 )

9.52 ( 1H, d, -CONH- )

Example 21

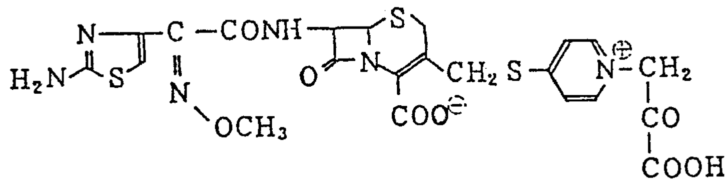

In 3.75 ml of N,N-dimethylformamide was dissolved 750 mg of (Z)-7-[α-(2-amino-4-thiazolyl)-α-(methoxyimino)acetamido]-3-(4-pyridyl)thiomethyl-$\Delta^3$-cephem-4-carboxylic acid-$H_2SO_4$ salt. Then 84.3 mg of potassium carbonate powder were added to the solution. After the mixture was stirred at room temperature for 30 minutes, the mixture was ice-cooled. After adding 934 μl of bistrimethylsilyl acetamide thereto, 608 mg of bromopyruvic acid was further added thereto under ice cooling. The mixture was stirred at room temperature for 24 hours. The reaction mixture was dispersed in 20 ml of isopropanol. The precipitated solid was taken by filtration and 740 mg of a pale yellow solid was obtained. The solid was dissolved in 5 ml of water and the solution was subjected to column chromatography using HP-20 (Diaion, manufactured by Mitsubishi Chemical Co., Ltd.), eluting firstly with water and then a 10% methanol-water mixture. Fractions containing the product were collected, concentrated and freeze dried to obtain 150 mg of (Z)-7-[α-(2-amino-4-thiazolyl)-α-(methoxyimino)acetamido]-3-(1-(2-carboxy-2-oxoethyl)-4-pyridinio)-thiomethyl-$\Delta^3$-cephem-4-carboxylate.

NMR (DMSO - $d_6$ )

δ;ppm

3.73 ( 3H, s, $-OCH_3$ )

4.51 ( 2H, q, $C_3-CH_2-$ )

5.02 ( 1H, d, $C_6-H$ )

5.55 ( 1H, dd, $C_7-H$ )

5.75 ( 2H, s, $>N^{\oplus}-CH_2-$ )

6.72 ( 1H, s, 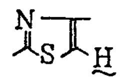 )

8.28 ( 2H, d, 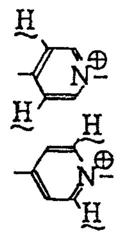 )

8.48 ( 2H, d, )

9.50 ( 1H, d, -CONH- )

Example 22

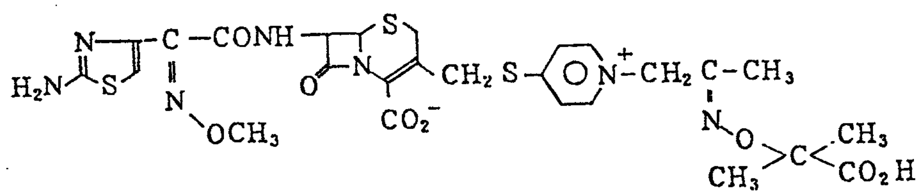

In 4 ml of N,N-dimethylformamide was dissolved 840 mg of (Z)-7-[α-(2-amino-4-thiazolyl)-α-(methoxyimino)acetamido]-3-(4-pyridyl)thiomethyl- $\Delta^3$-cephem-4-carboxylic acid-$H_2SO_4$ salt. Then 98 mg of potassium carbonate powder were added to the solution. After the mixture was stirred at room temperature for 30 minutes, the mixture was ice-cooled. After adding 1050 µl of N,O-bistrimethylsilyl acetamide thereto, the mixture was stirred at 0 to 5°C for 1.5 hours. Then, a solution of 1.01 g of 1-bromo-2-[1-carboxy-1-methylethoxyimino]propane and 349 µl of N,O-bistrimethylsilyl acetamide was added to the reaction mixture. The mixture was reacted at 4°C for 3

days.

The reaction solution was dispersed in 20 ml of isopropanol. The precipitated solid was taken by filtration, washed with isopropanol-ethyl acetate (1:1) and then with ethyl acetate and dried. The obtained powders were subjected to column chromatography using HP-20 and eluted with water : methanol of regularly changing methanol proportion. Fractions containing the product were collected, concentrated and freeze dried to obtain 270 mg of (Z)-7-[α-(2-amino-4-thiazolyl)-α-(methoxyimino)acetamido]-3-[[1-(2-(1-carboxy-1-methylethoxyimino)propyl]-4-pyridinio]thiomethyl- $\Delta^3$-cephem-4-carboxylate.

i) NMR ( $d^6$ - DMSO ) δ : ppm

1.24 ( 6H, s, 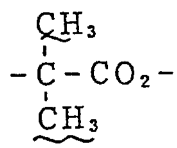 )

1.81 ( 3H, s, 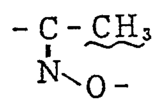 )

3.82 ( 3H, s, 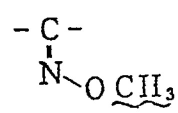 )

4.98 ( 1H, d, 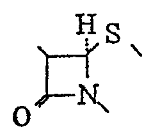 )

5.16 ( 2H, s, 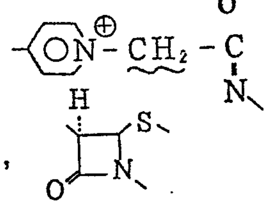 )

5.52 ( 1H, dd, )

6.79 ( 1H, s, 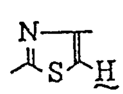 )

8.13 (2H, d, -S- 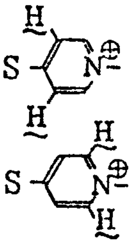 ) ;

8.53 (2H, d, -S- )

ii) IR(KBr) $cm^{-1}$

1770 (肩), 1755, 1660, 1615

iii) FABMS $[M+H]^+$

684

Reference Example 5:

(raw material for Examples 23 and 24)

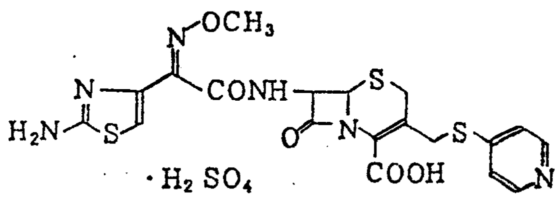

In 220 ml of dimethylformamide was dissolved 46.23 g of (Z)-α-(2-amino-4-thiazolyl)-α-methoxyiminoacetic acid and, 440 ml of 1,4-dioxane and 31.05 g of 1-hydroxybenzotriazole were added to the solution. The mixture was stirred to make it a homogeneous solution. Then 47.38 g of dicyclohexylcarbodiimide was added to the solution under ice cooling. After the mixture was stirred for 1 hour, dicyclohexyl urea precipitated was removed by filtration to obtain an activated ester - DMF/dioxane solution. On the other hand, 64.6 g of 7β-amino-3-(4-pyridyl)thiomethyl- $\Delta^3$-cephem-4-carboxylic acid

was added to 1300 ml of water and 14.78 g of lithium carbonate was further added thereto followed by stirring at room temperature. After insoluble matter in the resulting solution was removed by filtration, 1080 ml of dimethylformamide was added to the filtrate followed by cooling to +10°C. The activated ester - DMF/dioxane solution previously obtained was dropwise added thereto over 10 minutes and then stirred at about 5°C for 30 minutes. Thereafter 100 ml of 3N sulfuric acid was added to the reaction mixture. Insoluble matters were removed by filtration. The filtrate was washed with ethyl acetate 3 times (once with 1200 ml and twice with 800 ml). The aqueous phase was concentrated under reduced pressure and 1500 ml of acetone was added to the residue to make powders. The powders were dried under reduced pressure to obtain a crude product. While vigorously stirring, the crude product was gradually added to 200 ml of 3N sulfuric acid. After completion of the addition, the mixture was allowed to stand at room temperature for 40 minutes while gently stirring. Precipitated crystals were taken by filtration. After washing the crystals with 50 ml of water 4 times, 50 ml of acetone : water (1:1) 4 times and then 50 ml of acetone 4 times, in this order, the crystals were dried under reduced pressure to obtain 47.1 g (yield, 39%) of (Z)-7-[α-(2-amino-4-thiazolyl)-α-(methoxyimino)acetamido]-3-(4-pyridyl)thiomethyl-$\Delta^3$-cephem-4-carboxylic acid monosulfate.

The purity of the product was determined by HPLC, by the external standard, to be higher than 94.5%

NMR(DMSO-$d_6$/TMS)

δ : ppm 3.48, 3.78 (各1H, AB, J=18Hz, 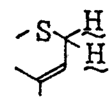 )

3.85 (3H, s, 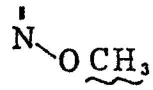 )

4.34 (2H, bs, 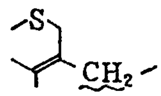 )

5.19 (1H, d, 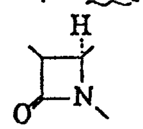 )

5.79 (1H, dd, J=4.6, 7.9Hz, 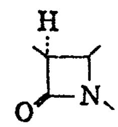 )

6.77 (1H, s, 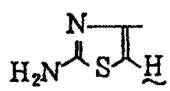 )

7.82 (2H, d, J=7Hz 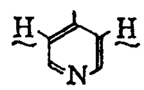 )

8.59 (2H, d, J=7Hz 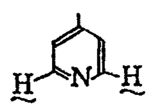 )

9.62 (1H, d, J=7.9Hz, -CONH- )

IR(KBr)

$\nu max$= 1775, 1621, 1120 $cm^{-1}$

Example 23

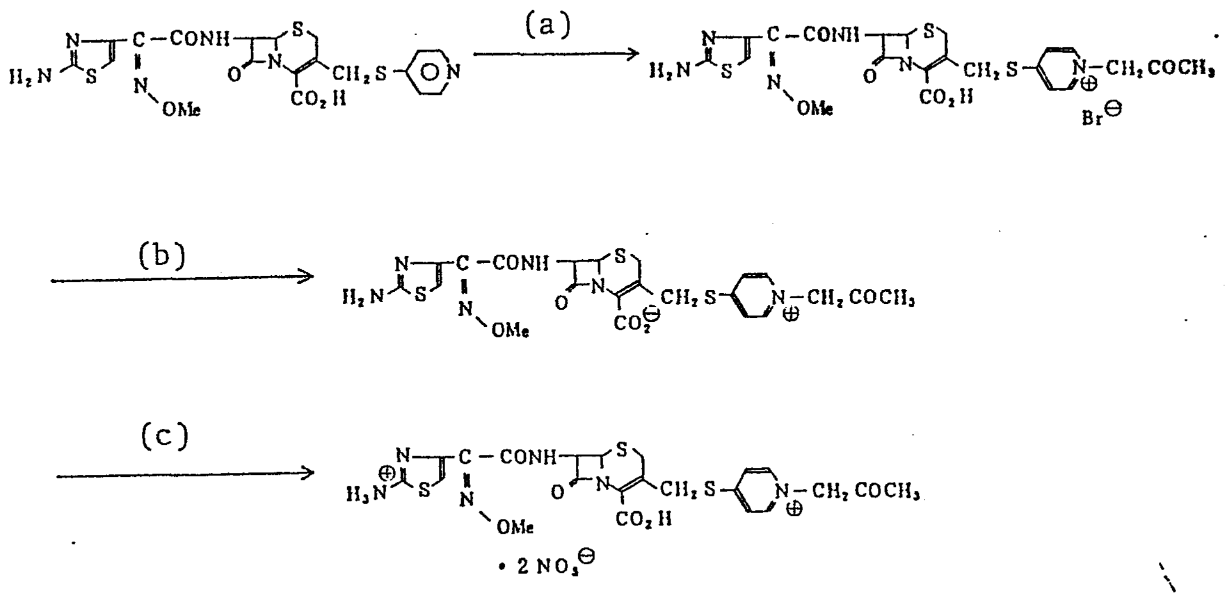


(a) In 50 ml of N,N-dimethylformamide was dissolved 10.0 g of (Z)-7-[α-(2-amino-4-thiazolyl)-α-(methoxyimino)acetamido]-3-(4-pyridyl)thiomethyl- $\Delta^3$-cephem-4-carboxylic acid $H_2SO_4$ salt. Then 1.16 g of potassium carbonate powder were added to the solution. After the mixture was stirred at room temperature for 30 minutes, the reaction mixture was cooled with ice.

After 12.4 ml of N,O-bistrimethylsilyl acetamide was dropwise added thereto, stirring was performed for 90 minutes under ice cooling. Then 4.3 ml of bromoacetone was added thereto . followed by stirring at 3°C for 18 hours. Thereafter 300 ml of isopropyl alcohol was added to the

mixture at 5°C or lower. After stirring the mixture for 2 hours under ice cooling, the formed precipitates were taken by filtration. The precipitates were washed with 100 ml of an isopropyl alcohol-ethyl acetate solvent mixture (1:1) and then with 50 ml of ethyl acetate, and dried over phosphorus pentachloride under reduced pressure to obtain 14.0 g of crude (Z)-7-[α-(2-amino-4-thiazolyl)-α-(methoxyimino)acetamido]-3-[1-acetonyl-4-pyridinio]thiomethyl-$\Delta^3$-cephem-4-carboxylate hydrobromide.

(b) This product was dissolved in 100 ml of water. The solution was subjected to column chromatography using HP-20 (Diaion, manufactured by Mitsubishi Chemical Industry Co., Ltd.) to desalt it. Thereafter the eluate was concentrated and freeze dried to obtain 8.4 g of (Z)-7-[α-(2-amino-4-thiazolyl)-α-(methoxyimino)acetamido]-3-[1-acetonyl-4-pyridinio)thiomethyl-$\Delta^3$-cephem-4-carboxylate.

(c) To 6.2 g of this compound were added 55 ml of water and then 45 ml of a 1N aqueous nitric acid solution to make a homogeneous solution. After adding seed crystals to the solution, the solution was stirred at room temperature for 2 hours and then under ice cooling for 2 hours and the precipitated crystals were then taken by filtration.

After washing the crystals with 6 ml of a 1N aqueous nitric acid solution cooled with ice and then with 18 ml of

isopropyl alcohol, the crystals were dried over phosphorus pentachloride under reduced pressure to obtain 6.4 g of (Z)-7-[α-(2-amino-4-thiazolyl)-α-(methoxyimino)acetamido]-3-[1-acetonyl-4-pyridinio)thiomethyl- $\Delta^3$-cephem-4-carboxylate nitrate [$2HNO_3$] crystals.

Example 24

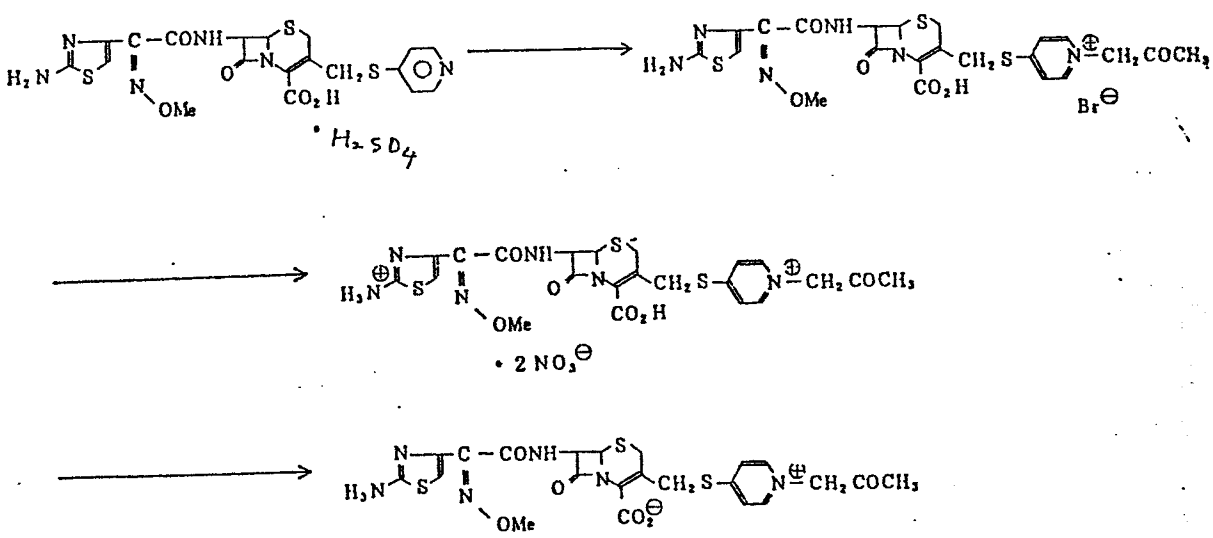

In 345 ml of N,N-dimethylformamide was dissolved 69.0 g of (Z)-7-[α-(2-amino-4-thiazolyl)-α-(methoxyimino)acetamido]-3-(4-pyridyl)thiomethyl- $\Delta^3$-cephem-4-carboxylic acid-$H_2SO_4$ salt. Then 7.76 g of potassium carbonate powder were added to the solution. After the mixture was stirred at room temperature for 30 minutes, the reaction mixture was cooled with ice.

After 85.9 ml of N,O-bistrimethylsilyl acetamide was dropwise added thereto over 10 minutes under ice cooling, stirring was performed for 90 minutes at the same temperature. Then 29.3 ml of bromoacetone was added thereto followed by stirring at 3°C for 16 hours.

After completion of the reaction, 2070 ml of isopropyl alcohol was added to the mixture at 5°C or lower. After stirring the mixture for 1 hour under ice cooling, the formed precipitates were taken by filtration. The precipitates were washed with 700 ml of an isopropyl alcohol-ethyl acetate solvent mixture (1:1) and then with 700 ml of ethyl acetate, and then dried over phosphorus pentachloride under reduced pressure to obtain 113 g of crude (Z)-7-[α-(2-amino-4-thiazolyl)-α-(methoxyimino)acetamido]-3-[1-acetonyl-4-pyridinio]-

thiomethyl- $\Delta^3$-cephem-4-carboxylate hydrobromide.

This crude product was dissolved in 685 ml of water. The solution was rendered neutral by adding 230 ml of 8 % aqueous sodium bicarbonate solution, to which 7.0 g of activated charcoal was added. The mixture was stirred for 5 minutes and then filtered. To the filtrate were added 469 ml of 1N aqueous nitric acid solution and then 7.0 g of activated charcoal. The mixture was filtered.

After adding seed crystals to the filtrate, the mixture was stirred at room temperature for 3 hours and at 3°C for 16 hours. The precipitated crystals were taken by filtration. After washing the crystals with 200 ml of a 1N aqueous nitric acid solution and then with 150 ml of isopropyl alcohol, the crystals were dried over phosphorus pentachloride under reduced pressure to obtain 45.4 g of (Z)-7-[α-(2-amino-4-thiazolyl)-α-(methoxyimino)acetamido]-3-[1-acetonyl-4-pyridinio]thiomethyl- $\Delta^3$-cephem-4-carboxylate dinitrate.

The dinitrate, 45.0 g, was dissolved in 1.5 liters of water. An aqueous potassium carbonate solution was added to

the solution to adjust pH to 3. Then the solution was subjected to column chromatography using HP-20 (Diaion, manufactured by Mitsubishi Chemical Industry Co., Ltd.). After eluting it with ion exchange water to desalt it, the desired product was eluted with a 70% methanol-water mixture. Thereafter the eluate was concentrated and then freeze dried to obtain 34.9 g of (Z)-7-[α-(2-amino-4-thiazolyl)-α-(methoxyimino)acetamido]-3-[1-acetonyl-4-pyridinio)thiomethyl- $\Delta^3$-cephem-4-carboxylate.

Reference Example 6 (raw material of Examples 25 to 30)

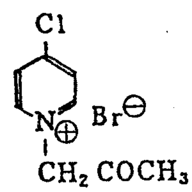

(a) To a solution of 200.0 g of 4-chloropyridine hydrochloride in 500 ml of water was added 1500 ml of ether. Under ice cooling, 141.2 g of sodium carbonate was added to the mixture under ice cooling. The organic phase was isolated and washed with a saturated aqueous sodium chloride solution. Thereafter magnesium sulfate was added to the organic phase The solution was concentrated under reduced pressure to obtain 141.8 g of an oily substance. Under ice cooling 205.4 g of bromoacetone was added to a solution of the oily substance in 1000 ml of ether. The mixture was stirred at the same temperature for 4 days. The

formed precipitates were taken by filtration, washed with ether and then dried to obtain 113.6 g of 1-acetonyl-4-chloropyridinium bromide.

NMR (DMSO - $d_6$)

$\delta$ : ppm 2.32 ( 3 H, s, $-COCH_3$ )

5.78 ( 2 H, s, $\rangle N^+ - CH_2 - CO -$ )

8.43 ( 2 H, d, J=8.0 Hz, 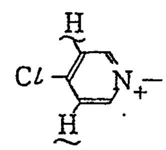 )

8.90 ( 2 H, d, J=8.0 Hz, 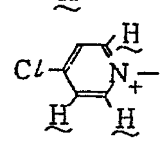 )

(b)

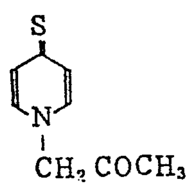

(b) A solution of 24.00 g of sodium sulfide 9 hydrate in 50 ml of water was added to a solution of 25.05 g of 1-acetonyl-4-chloropyridinium bromide in 75 ml of water under ice cooling, while stirring. Stirring was conducted for 30 minutes under ice cooling. The formed crystals were taken out by filtration, washed with chilled water and then dried to obtain 12.24 g of 1-acetonyl-4-thiopyridone.

0190900

Elemental analysis: $C_8H_9NOS$:

Calculated: C 57.46, H 5.42, N 8.38, S 19.17

Found: C 57.43, H 5.38, N 8.25, S 18.93

NMR ( DMSO - $d_6$ )

$\delta$ : ppm 2.17 ( 3 H, s, -COC$\underset{\sim}{H_3}$ )

5.04 ( 2 H, s, 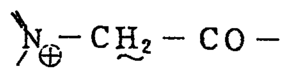 )

7.15 ( 2 H, d, J=8.0 Hz, 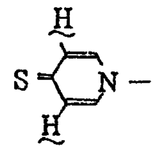 )

7.38 ( 2 H, d, J=8.0 Hz, 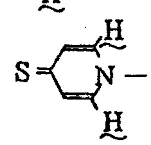 )

m.p 163 - 4 ℃

Example 25

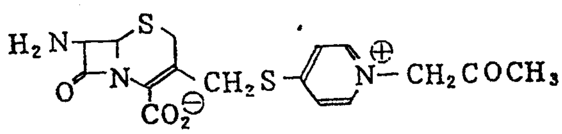

A mixture of 810 mg of 7-aminocephalosporanic acid, 1.0 g of 1-acetonyl-4-thiopyridone, 10.0 g of potassium

iodide, 252 mg of sodium hydrogen carbonate and 5 ml of water was stirred at 53 to 55°C for 4 hours. The reaction mixture was cooled, diluted with water and subjected to column chromatography using Diaion HP-20. After eluting with water, the desired product was eluted with 20% aqueous methanol. The eluates were collected, concentrated under reduced pressure, and freeze dried to obtain 402 mg of 7-amino-3-(1-acetonyl-4-pyridinio)thiomethyl)-$\Delta^3$-cephem-4-carboxylate.

NMR ( DMSO $-d_6+D_2O$ )

δ : ppm 2.30 ( 3 H, s, $-CH_2-\overset{O}{\overset{\|}{C}}\underset{\sim}{CH_3}$ )

3.48 ( 2 H, q, 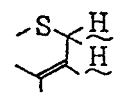 )

4.52 ( 2 H, q, 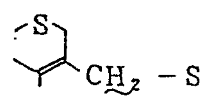 )

4.59 ( 1 H, d, 6位 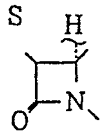 )

4.88 ( 1 H, d, 7位 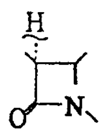 )

8.26 ( 2 H, d, 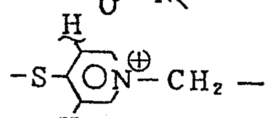 )

8.48 ( 2 H, d, 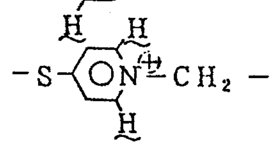 )

(b)

0190900

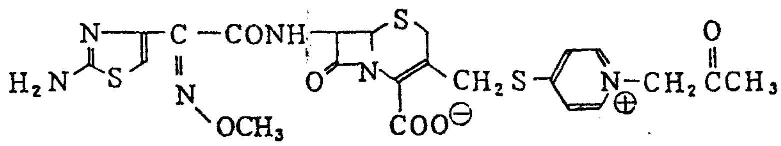

A mixture of 544 mg of 7-aminocephalosporanic acid, 667 mg of 1-acetonyl-4-thiopyridone, 3.33 mg of potassium iodide, 168 mg of sodium hydrogen carbonate and 4 ml of water was stirred for 4 hours at 52°C; (the formed solution is called "Solution A").

On the other hand, 402 mg of (Z)-α-(2-amino-4-thiazolyl)-α-(methoxyimino)acetic acid and 270 mg of 1-hydroxybenzotriazole were dissolved in a mixture of 2 ml of dimethylformamide and 4 ml of 1,4-dioxane and after adding thereto 412 mg of dicyclohexylcarbodiimide, the resultant mixture was stirred for 30 minutes at room temperature. Precipitated dicyclohexylcarbodiimide was removed by filtration to give a soltuion of an active ester (the formed solution is called "Solution B").

After cooling Solution A and adding thereto Solution B dropwise, the mixture was stirred for 2 hours at room temperature. To the reaction solution was added water and after washing the solution with ethyl acetate twice, the aqueous layer was concentrated under reduced pressure to remove organic solvents. The residue was diluted with water and subjected to column chromatography on Diaion HP-20. After eluting with water, the product was eluted with mixed solutions of water and methanol, successively changing the mixing ratio. The fractions containing the desired product were collected, concentrated, and lyophilized to provide 500 mg of (Z)-7-[α-(2-amino-4-thiazolyl)-α-(methoxyimino)acetamido]-3-[1-acetonyl-4-pyridinio]thiomethyl-$\Delta^3$-cephem-4-carboxylate. The physico-chemical characteristics of this product coincide with those of the compound of Example 4.

Example 26

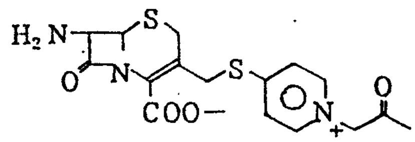

(a) To a solution of 1.90 g of 7-aminocephalosporanic acid and 1.29 g of 1-acetonyl-4-thiopyridone in 15 ml of nitromethane was added 1.62 ml of fluorosulfonic acid under ice cooling, while stirring. The mixture was stirred for a further 4 hours at the same temperature. After 1.01 ml of water was added to the mixture and the system was stirred for 30 minutes under ice cooling, acetone was added thereto. The formed powders were filtered and dried to obtain 3.94 g of a crude product.

(b) A part (500 mg) of the crude product was dissolved in 30 ml of water. After the pH was adjusted to 5 with sodium hydrogen carbonate, the solution was subjected to column chromatography using Diaion HP-20 and eluted firstly with water and then a 10-20 % methanol solution. Fractions containing the product were collected, concentrated and freeze dried to obtain 266 mg of 7-amino-3-(1-acetonyl-4-pyridinio)thiomethyl-$\Delta^3$-cephem-4-carboxylate.

The physico-chemical characteristics (e.g. NMR, etc.) of this product coincide with those of the compound of Ex. 29(a).

(c)

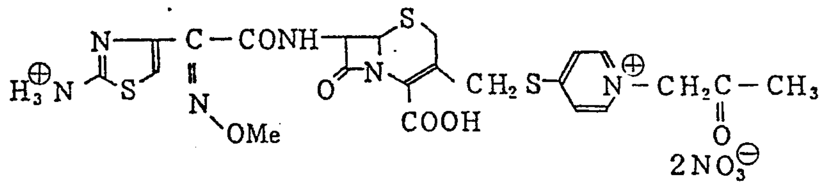

In 30 ml of water and 10 ml of tetrahydrofuran was suspended 2.88 g of the crude product obtained in (a). Under ice cooling, 1.68 g of sodium hydrogen carbonate was added to the suspension to dissolve (Solution A).

On the other hand, 1.21 g of (Z)-α-(2-amino-4-thiazolyl)-α-(methoxyimino)acetic acid and 810 mg of 1-hydroxybenzotriazole were added to 4.4 ml of dimethylformamide and 7.2 ml of tetrahydrofuran. Thereafter 1.24 g of dicyclohexylcarbodiimide was added to the mixture. The resulting mixture was stirred at room temperature for 30 minutes. Dicyclohexyl urea formed was removed by filtration to obtain a solution of the activated ester (Solution B).

After Solution A was cooled, Solution B was dropwise added thereto. The mixture was stirred at room temperature for 2 ½ hours. To the reaction mixture was added 30 ml of water. The mixture was washed with 60 ml of dichloromethane twice, and 1.45 ml of concentrated nitric acid and 30 ml of water were added thereto. The solution was treated with 280 mg of activated charcoal. The solution was concentrated until syrup-like matter was

precipitated. By stirring the concentrate under ice cooling, crystals of (Z)-7-[α-(2-amino-4-thiazolyl)-α-(methoxyimino)-acetamido]-3-[1-acetonyl-4-pyridinio]thiomethyl-$\Delta^3$-cephem-4-carboxylate dinitrate were precipitated. The crystals were taken out by filtration, washed with a chilled 1N nitric acid aqueous solution and then with cold ethanol and finally dried under reduced pressure to obtain 1.38 g of the product.

NMR (DMSO - $d_6$)

δ:ppm 2.27 (3H, s, $-CH_2COC\underline{H_3}$)

3.67 (2H, q, 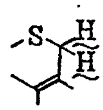 )

3.93 (3H, s, $=N-O-C\underline{H_3}$ )

4.40 (2H, s, 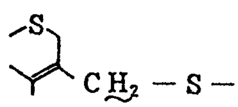 )

5.23 (1H, d, 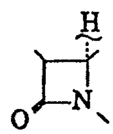 )

5.58 (2H, s, $-C\underline{H_2}-COCH_3$ )

5.80 (1H, dd, 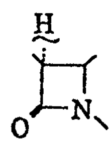 )

6.91 (1H, s, 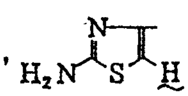 )

8.04 (2H, d, 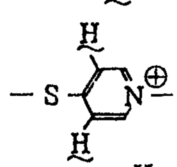 )

8.49 (2H, d, 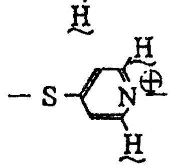 )

Example 27

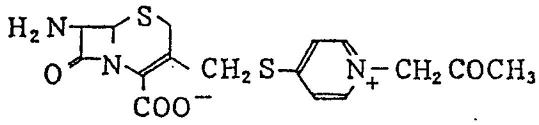

(a) To a solution of 1.63 g of 7-aminocephalosporanic acid and 1.20 g of 1-acetonyl-4-thiopyridone in 20 ml of nitromethane was added 1.59 ml of chlorosulfonic acid at -10°C, while stirring. The mixture was stirred for a further 1.5 hours under ice cooling. After 0.86 ml of water was added to the mixture, the system was stirred for a further 30 minutes under ice cooling. Then acetone was added to the reaction mixture. The formed powders were taken by filtration, washed with acetone and dried to obtain 3.87 g of a crude product.

(b) A part (500 mg) of the crude product was dissolved in 30 ml of water. After the pH was adjusted to 5 with sodium hydrogen carbonate, the solution was subjected to column chromatography using Diaion HP-20 and eluted firstly with water and then with an aqueous solution containing 10 - 20 % methanol. Fractions containing the product were collected, concentrated

and freeze dried to obtain 197 mg of 7-amino-3-(1-acetonyl-4-pyridinio)thiomethyl- $\Delta^3$-cephem-4-carboxylate.

(c) In 30 ml of water and 10 ml of tetrahydrofuran was suspended 2.88 g of the crude product obtained in (a). Under ice cooling, 1.68 mg of sodium hydrogen carbonate was added to the suspension to dissolve (Solution A).

On the other hand, 1.21 g of (Z)-α-(2-amino-4-thiazolyl)-α-(methoxyimino)acetic acid and 810 mg of 1-hydroxybenzotriazole were added to 4.4 ml of dimethylformamide and 7.2 ml of tetrahydrofuran. Thereafter 1.24 g of dicyclohexylcarbodiimide was added to the mixture. The resulting mixture was stirred at room temperature for 30 minutes. Dicyclohexyl urea formed was removed by filtration to obtain a solution of the activated ester (Solution B).

After Solution A was cooled, Solution B was dropwise added thereto. The mixture was stirred at room temperature for 2½ hours. To the reaction mixture was added 30

ml of water. The mixture was washed with 60 ml of dichloromethane twice, and 1.45 ml of concentrated nitric acid and 30 ml of water were added thereto. The mixture was decolored with 280 mg of activated charcoal. The solution was concentrated until thick syrup-like matter was precipitated. By stirring the concentrate under ice cooling, crystals of (Z)-7-[α-(2-amino-4-thiazolyl)-α-(methoxyimino)-acetamido]-3-[1-acetonyl-4-pyridinio]thiomethyl-$\Delta^3$-cephem-4-carboxylate dinitrate were precipitated. The crystals were taken out by filtration, washed with a chilled 1N nitric acid aqueous solution and then with cold ethanol and finally dried under reduced pressure to obtain 1.38 g of the product.

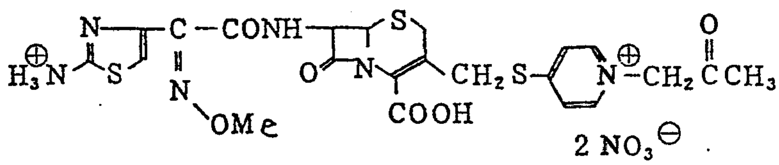

Example 28

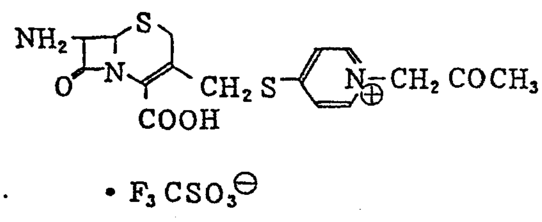

(a) To a solution of 2.72 g of 7-aminocephalosporanic acid and 1.84 g of 1-acetonyl-4-thiopyridone in 30 ml of nitromethane was added 3.54 ml of trifluoromethanesulfonic acid at -20°C, while stirring. The mixture was stirred for a further 30 minutes at the same temperature and further under

 

ice cooling for 3 hours. After 100 ml of isopropyl alcohol was added to the reaction mixture, 4.17 ml of triethyl amine was further added thereto under ice cooling. The formed precipitates were taken by filtration, washed with isopropyl alcohol and dried to obtain 4.91 g of 7-amino-3-(1-acetonyl-4-pyridinio)thiomethyl-$\Delta^3$-cephem-4-carboxylate trifluoromethanesulfonate($CF_3SO_3H$ salt).

NMR (DMSO-$d_6$)

δ : ppm 2.28 (3H, s, $-COC\underset{\sim}{H_3}$ )

3.65 (2H, ABq, 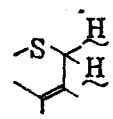 )

4.38 (2H, s, 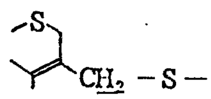 )

4.90 (1H, d, J=6Hz, 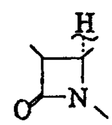 )

5.09 (1H, d, J=6Hz, 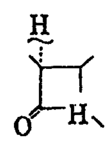 )

5.53 (2H, s, $\overset{+}{N}-C\underset{\sim}{H_2}-CO-$ )

8.04 (2H, d, J=8Hz, 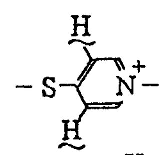 )

8.46 (2H, d, J=8Hz, 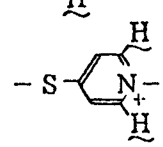 )

(b) In 13.3 ml of water and 5 ml of tetrahydrofuran was suspended 1.325 g of 7-amino-3-(1-acetonyl-4-pyridinio)-thiomethyl- $\Delta^3$-cephem-4-carboxylate trifluoromethanesulfonate. Under ice cooling, 420 mg of sodium hydrogen carbonate was added to the suspension to dissolve (Solution A).

On the other hand, 604 mg of (Z)-α-(2-amino-4-thiazolyl)-α-(methoxyimino)acetic acid and 406 mg of 1-hydroxybenzotriazole were added to 2.2 ml of dimethylformamide and 3.6 ml of tetrahydrofuran. Thereafter 620 mg of dicyclohexylcarbodiimide was added to the mixture under ice cooling. The resulting mixture was stirred at the same temperature for 1½ hours. Dicyclohexyl urea formed was removed by filtration to obtain a solution of the activated ester (Solution B).

After Solution A was cooled, Solution B was dropwise added thereto. The mixture was stirred overnight under ice cooling. To the reaction mixture was added 1.7 ml of water. The mixture was washed with 20 ml of dichloromethane twice. Then, 724 μl of conc. nitric acid and 25 ml of water were added thereto and the mixture was decolored with 200 mg of activated charcoal. The solution was concentrated until thick syrup-like matter was precipitated. By stirring the concentrate under ice cooling, crystals of (Z)-7-[α-(2-amino-4-thiazolyl)-α-(methoxyimino)acetamido]-3-[1-acetonyl-4-pyridinio]thiomethyl- $\Delta^3$-cephem-4-carboxylate dinitrate were precipitated. The crystals were taken out by filtration, washed with a chilled 1N nitric acid aqueous solution and then with cold ethanol and finally dried under reduced pressure to obtain 788 mg of the product. Its NMR etc. coincide with Ex. 26(c).

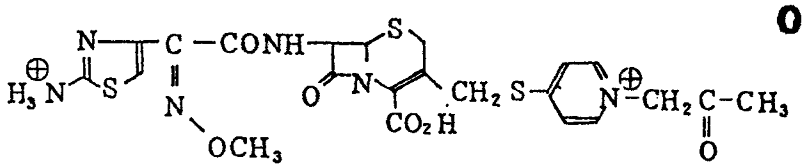

$2\ NO_3^{\ominus}$

Example 29

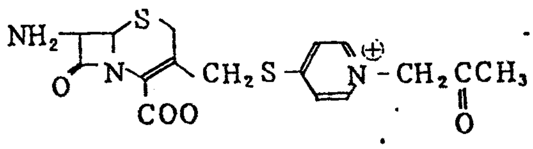

(a) In 30 ml of dichloromethane was suspended 1.02 g of 7-amino-3-iodomethyl-3-cephem-4-carboxylic acid and 2.0 ml of O,N-bis(trimethylsilyl)trifluoroacetamide was added to the suspension. The suspension was stirred at room temperature for 30 minutes to dissolve. To the solution was added 0.5 g of 1-acetonyl-4-thiopyridone and then 1 ml of O,N-bis(trimethylsilyl)trifluoroacetamide to make a homogeneous solution. After the solution was stirred at room temperature for 1 hour, 2 ml of 1,3-butanediol was added thereto. The formed precipitates were filtered, washed with 10 ml of dichloromethane and then dried to obtain 1.2 g of a crude product. The crude product was dissolved in 150 ml of water. After insoluble matters were filtered off using perlite, the filtrate was treated with Diaion HP-20 (manufactuared by Mitsubishi Chemical Industry Co., Ltd.). Eluates obtained by eluting with water and methanol in mixing ratios changing from 9:1 to 8:2 were concentrated and freeze dried to obtain 370 mg of 7-amino-3-(1-acetonyl-4-pyridinio)thiomethyl-$\Delta^3$-cephem-4-carboxylate.

NMR ($D_2O$)

ppm 2.39 (3H, s, 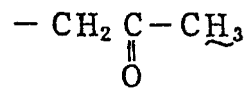 )

3.56 (2H, q, 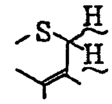 )

4.31 (2H, q, 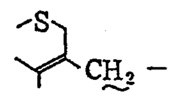 )

4.79 (2H, d, 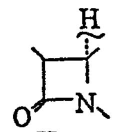 )

5.03 (2H, d, 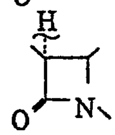 )

7.84 (2H, d, 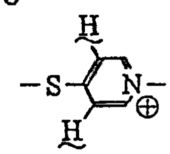 )

8.25 (2H, d, 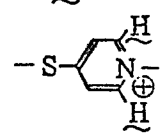 )

(b)

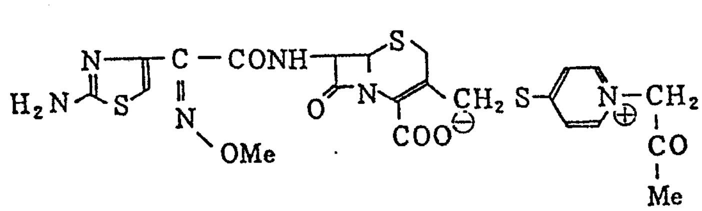

In 12 ml of water was dissolved 360 mg of 7-amino-(1-acetonyl-4-pyridinio)thiomethyl-$\Delta^3$-cephem-4-carboxylate under ice cooling (Solution A).

On the other hand, 20.1 g of (Z)-α-(2-amino-4-thiazolyl)-α-(methoxyimino)acetic acid and 13.5 g of 1-hydroxybenzotriazole were dissolved in 100 ml of dimethylformamide. Under ice cooling 22.5 g of dicyclohexylcarbodiimide was added to the solution. After the mixture was stirred at room temperature for 1 hour, dicyclohexyl urea formed was removed by filtration and 200 ml of ethyl acetate was added to the filtrate. The resulting solution was washed with 200 ml of water twice, dried over magnesium sulfate and concentrated. Ether, 100 ml, was added to the residue to make powders. The powders were filtered and dried to obtain 8 g, from which 362 mg was taken and dissolved in 3 ml of dimethylformamide. The solution was added to Solution A. The mixture was stirred at room temperature for 1 hour. After the reaction mixture was concentrated, 150 ml of water was added to the concentrate. Insoluble matters were filtered off using perlite. The filtrate was treated with Diaion HP-20 and fractions eluted with water : methanol (7:3) were concentrated and freeze dried to obtain 350 mg of (Z)-7-[α-(2-amino-4-thiazolyl)-α-(methoxyimino)acetamido]-3-[1-acetonyl-4-pyridinio]thiomethyl-$\Delta^3$-cephem-4-carboxylate.
The physico-chemical characteristics (NMR, etc.) of this product coincide with those of the compound of Example 4.

Example 30

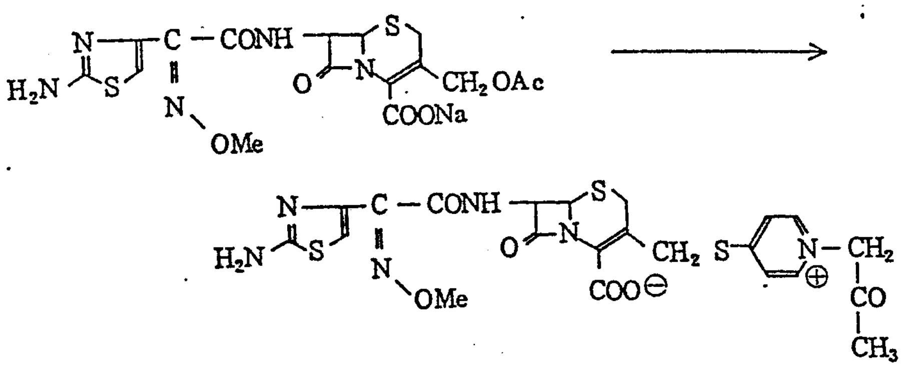

In 8 ml of water was dissolved 1.9 g of sodium (Z)-7-[α-(2-amino-4-thiazolyl)-α-(methoxyimino)acetamido]-3-acetoxy-$\Delta^3$-cephem-4-carboxylate. To the solution were added 1 g of 1-acetonyl-4-thiopyridone and 13.28 g of potassium iodide. The mixture was stirred for 5 hours at the inner temperature of 57 to 58°C. The reaction solution was dissolved in 100 ml of water and the solution was treated with 150 ml of Diaion HP-20. Fractions obtained by eluting with water : methanol (8:2) were concentrated and freeze dried to obtain 0.93 g of (Z)-7-[α-(2-amino-4-thiazolyl)-α-(methoxyimino)acetamido]-3-[1-acetonyl-4-pyridinio]thiomethyl-$\Delta^3$-cephem-4-carboxylate. Its NMR etc. coincide with Ex. 4.

Example 31 (Preparation of the nitrate crystals)

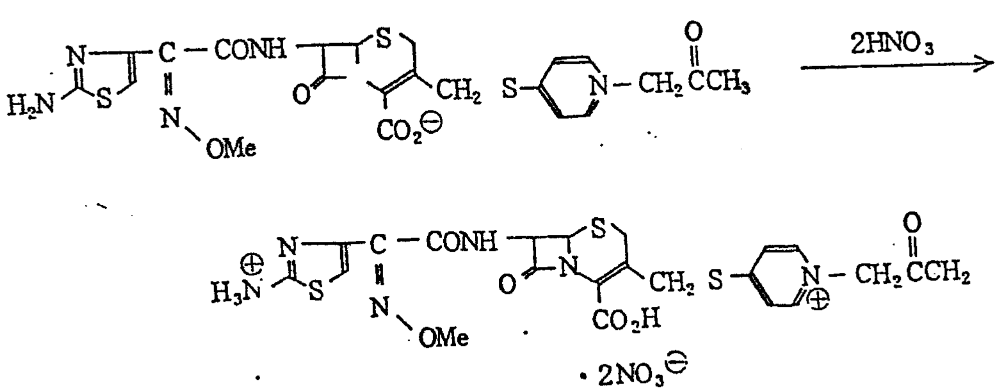


In 240 ml of water was suspended 24.9 g of (Z)-7-[α-

 

(2-amino-4-thiazolyl)-α-(methoxyimino)acetamido]-3-[1-acetonyl-4-pyridinio]thiomethyl- $\Delta^3$-cephem-4-carboxylate. Then 180 ml of a 1N nitric acid aqueous solution was dropwise added to the suspension over 10 minutes while stirring. After adding seed crystals, the system was stirred at room temperature for 3 hours and for 1 hour under ice cooling. The system was then filtered.

The crystals taken by filtration were washed with 20 ml of a 1N nitric acid aqueous solution and then with 60 ml of isopropyl alcohol and dried over phosphorus pentachloride under reduced pressure to obtain 25.0 g of (Z)-7-[α-(2-amino-4-thiazolyl)-α-(methoxyimino)acetamido]-3-[1-acetonyl-4-pyridinio]thiomethyl- $\Delta^3$-cephem-4-carboxylate dinitrate.

NMR

δ ; ppm 2.28 (3H, s, 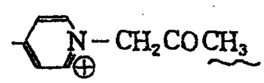

3.51, 3.83 (1H, 1H, AB, J=18Hz,

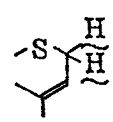

3.94 (3H, s, 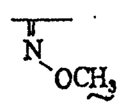 )

4.41 (2H, br, 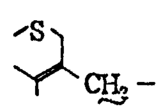 )

5.23 (1H, d, 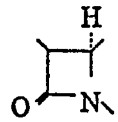 )

5.57 (2H, br, 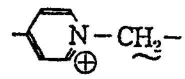 )

5.81 (1H, dd, 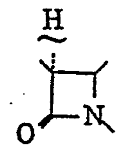 )

6.92 (1H, s, 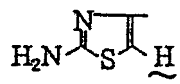 )

8.05 (2H, d, J = 6.8 Hz 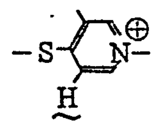 )

8.51 (2H, d, J = 6.8 Hz 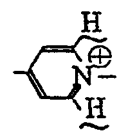 )

9.78 (1H, d, J = 7.9 Hz −CONH−

IR (KBr)

3280 $cm^{-1}$, 3050, 1762 $cm^{-1}$, 1718 $cm^{-1}$,

1650 $cm^{-1}$, 1624 $cm^{-1}$, 1380 $cm^{-1}$

Elemental analysis ($C_{22}H_{22}N_6O_6S_3 \cdot 2HNO_3 \cdot 1H_2O$)

| | C(%) | H(%) | N(%) | S(%) |
|---|---|---|---|---|
| Found: | 37.14 | 3.55 | 15.59 | 13.48 |
| Calculated: | 37.39 | 3.71 | 15.86 | 13.61 |

Example 32 (Preparation of the sulfate crystals)

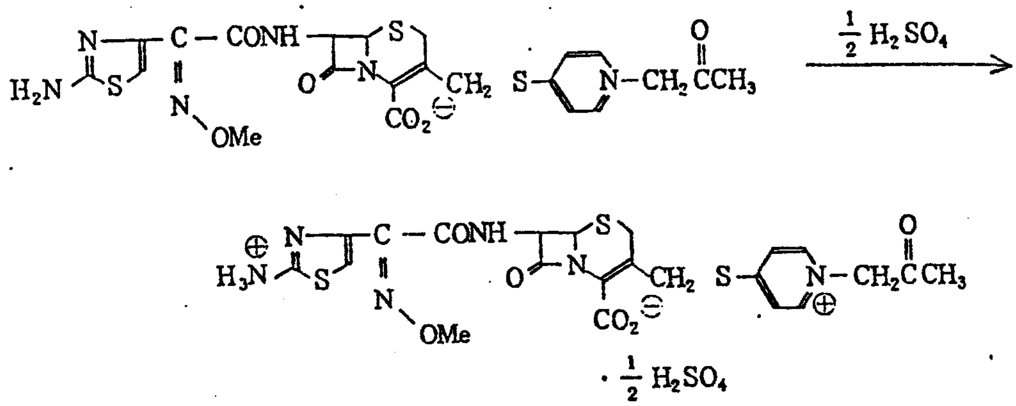


In 9.5 ml of water was suspended 2.0 g of (Z)-7-[α-(2-amino-4-thiazolyl)-α-(methoxyimino)acetamido]-3-[1-acetonyl-4-pyridinio]thiomethyl-$\Delta^3$-cephem-4-carboxylate. Then 0.52 ml of a 6.4N sulfuric acid aqueous solution was added to the suspension.

After adding seed crystals, the system was stirred at room temperature for 10 minutes and then settled at 3°C for 16 hours. The precipitated crystals were taken by filtration, washed with 70% ethanol-water and dried over phosphorus pentachloride under reduced pressure to obtain 1.52 g of (Z)-7-[α-(2-amino-4-thiazolyl)-α-(methoxyimino)-acetamido]-3-[1-acetonyl-4-pyridinio]thiomethyl-$\Delta^3$-cephem-4-carboxylate hemisulfate.

NMR

δ ; ppm 2.26 (3H, s, 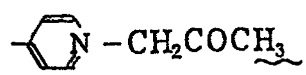 )

2.6 (2H, q, 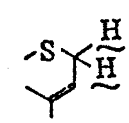 )

3.82 (3H, s, 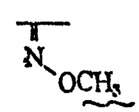 )

4.44 (2H, br, 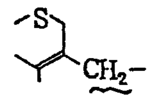 )

5.12 (1H, d, 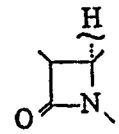 )

5.56 (2H, br, 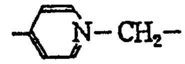 )

5.70 (1H, dd, 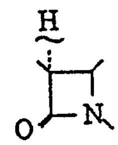 )

6.72 (1H, s, 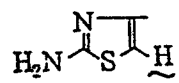 )

7.18 (2H, br, 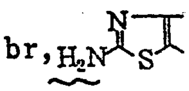 )

8.14 (2H, d, 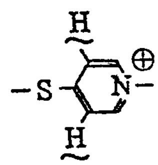 )

8.48 (2H, d, 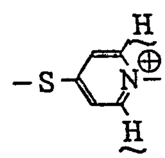 )

9.54 (1H, d, $-CONH-$ )

IR (KBr)

1756 $cm^{-1}$, 1720 $cm^{-1}$, 1645 $cm^{-1}$, 1620 $cm^{-1}$,
1350 $cm^{-1}$

Elemental analysis: ( $C_{22}H_{22}N_6O_6S_3 \cdot \frac{1}{2} H_2SO_4 \cdot 2H_2O$ )

| | C (%) | H (%) | N (%) | S (%) |
|---|---|---|---|---|
| Found: | 41.07 | 4.20 | 12.86 | 17.25 |
| Calculated: | 40.80 | 4.20 | 12.98 | 17.33 |

Example 33

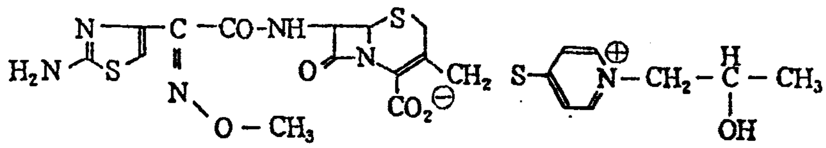

Under ice cooling 112 mg of (Z)-7-[α-(2-amino-4-thiazolyl)-α-(methoxyimino)acetamido]-3-[1-acetonyl-4-pyridinio]-thiomethyl-$\Delta^3$-cephem-4-carboxylate was dissolved in 2.2 ml of water and 2.2 ml of methanol and, 75 mg of primary celium chloride 7 hydrate was added to the solution followed by stirring. To the solution was added 8 mg of sodium borohydride. The mixture was stirred for 30 minutes at the same temperature. Then 0.5 ml of 1N hydrochloric acid was added thereto to adjust the pH to 2. Methanol was removed by distillation at low temperature under reduced pressure. Water was added to the residue. The aqueous phase was subjected to column chromatography using Diaion HP-20, eluting firstly with water and then with a water-methanol mixture. Fractions containing the product were collected, concentrated and freeze dried to obtain 70 mg of (Z)-7-[α-(2-amino-4-thiazolyl)-α-(methoxyimino)acetamido]-3-[1-(2-hydroxypropyl)-4-pyridinio]thiomethyl- $\Delta^3$-cephem-4-carboxylate.

NMR (DMSO-$d_6$)

δ : ppm 1.14 (3H, d, 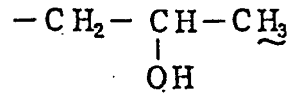 )

3.80 (3H, s, 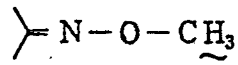 )

4.47 (2H, q, 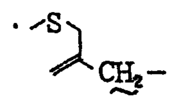 )

5.01 (1H, d, 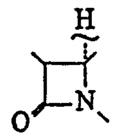 )

5.54 (1H, dd, 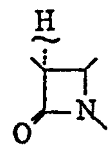 )

6.70 (1H, s, 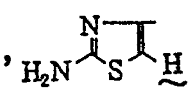 )

8.26 (2H, d, 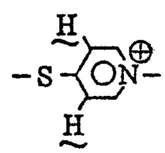 )

8.64 (2H, d, 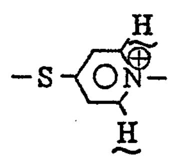 )

9.50 (1H, d, 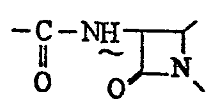 )

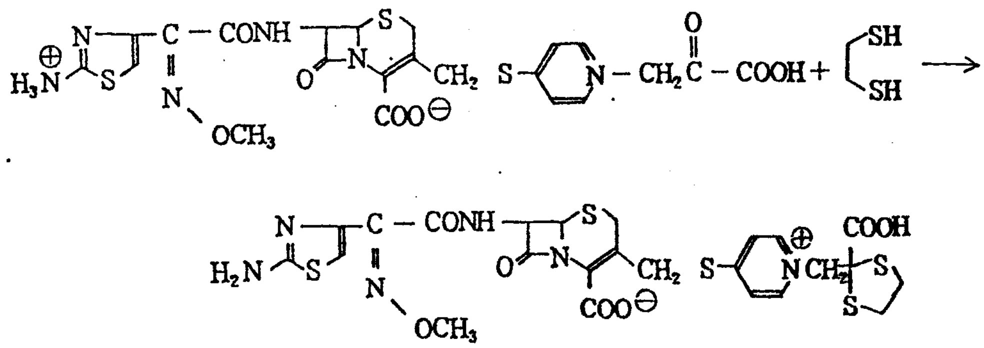

In 5 ml of dimethylformamide was dissolved 395 mg of (Z)-7-[α-(2-amino-4-thiazolyl)-α-(methoxyimino)acetamido]-3-[1-(2-carboxy-2-oxoethyl)-4-pyridinio]thiomethyl- $\Delta^3$-cephem-4-carboxylate. Then 120 μl of 1,2-ethanedithiol and 120 μl of borontrifluoride ethyl ether complex were added to the solution under ice cooling. Thereafter, the mixture was stirred at room temperature overnight. The reaction mixture was dispersed in 50 ml of isopropyl alcohol. The precipitated solid was taken by filtration, thoroughly washed firstly with 10 ml of water-methanol (1:1 in a volume ratio) and then with 10 ml of water and dried over phosphorus pentachloride under reduced pressure to obtain 324 mg of (Z)-7-[α-(2-amino-4-thiazolyl)-α-(methoxyimino)acetamido]-3-[1-[2-carboxy-1,3-dithiolan-2-yl)methyl]-4-pyridinio]-thiomethyl- $\Delta^3$-cephem-4-carboxylate.

NMR ($d_6$ - DMSO)

δ : ppm 2.6 ~ 3.1 ( 4 H, m 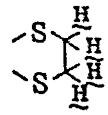 )

3.5 6 ( 2 H, q, $C_2<^{H}_{H}$ )

3.8 3 ( 3 H, s, $-OCH_3$ )

4.3 5 ( 2 H, s, $C_3-CH_2-$ )

5.1 6 ( 1 H, d, $C_6-H$ )

5.7 7 ( 1 H, dd, $C_7-H$ )

6.7 2 ( 1 H, s, 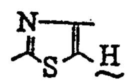 )

7.9 8 ( 2 H, d, 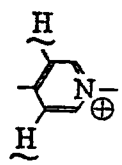 )

8.4 4 ( 2 H, d, 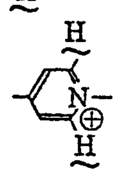 )

9.5 8 ( 1 H, d, $-CONH-$ )

Example 35

In 3.7 ml of N,N-dimethylformamide was dissolved 750 mg of (Z)-7-[α-(2-amino-4-thiazolyl)-α-(methoxyimino)acetamido]-3-(4-pyridyl)thiomethyl- $\Delta^3$-cephem-4-carboxylate sulfate. Then 84 mg of potassium carbonate powder were added to the solution. After the mixture was stirred at room temperature for 30 minutes, the reaction mixture was cooled with ice. After 934 µl of N,O-bistrimethylsilyl acetamide was added to the system, stirring was performed for 10 minutes at 0 to 5°C. Then 718 mg of 1-bromo-2-methoxyimino-propionic acid was added to the system followed by stirring at room temperature overnight. The reaction mixture was dispersed in 30 ml of isopropanol. The precipitated solid was taken by filtration. The solid was washed with 10 ml of isopropanol and 10 ml of ethyl ether twice and then dried. The obtained powders were subjected to columnn chromatography using HP-20 and eluted with water-methanol of regularly changing methanol proportion. Fractions containing the product were collected, concentrated and freeze dried to obtain 276 mg of (Z)-7-[α-(2-amino-4-thiazolyl)-α-(methoxyimino)acetamido]-[1-[2-methoxyimino)propionic acid]-4-pyridinio]thiomethyl- $\Delta^3$-cephem-4-carboxylate.

Example 36

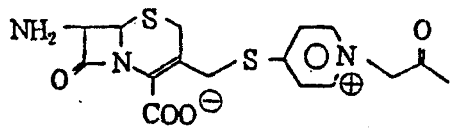

To a solution of 136 mg of 7-aminocephalosporanic acid and 92 mg of 1-acetonyl-4-thiopyridone in 1.0 ml of acetic acid was added 284 mg of boron trifluoride ethyl ether complex. The mixture was stirred at room temperature for 7 days. The solvent was removed from the reaction mixture by distillation and the residue was dissolved in 20 ml of water. After the pH was adjusted to 5 with sodium hydrogen carbonate, the system was subjected to column chromatography using Diaion HP-20 and eluted firstly with water and then with a 10-20 % methanol solution. Fractions containing the product were collected, concentrated and freeze dried to obtain 82 mg of 7-amino-3-(1-acetonyl-4-pyridinio)thiomethyl-$\Delta^3$-cephem-4-carboxylate.

By using this product as starting material and following the same procedure as in Example 29(b), (Z)-7-[α-(2-amino-4-thiazolyl)-α-(methoxyimino)acetamido]-3-[1-acetonyl-4-pyridinio]thiomethyl-$\Delta^3$-cephem-4-carboxylate was obtained.

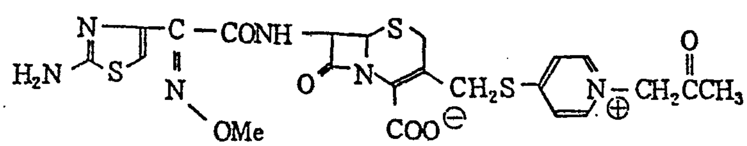

Example of Formulation:

After 0.5 g (titer) of the compound obtained in Example 31 and 0.1 g of anhydrous sodium carbonate were aseptically packed in a vial having an inner volume of 20 ml, the system was sealed under reduced pressure and fastened with an aluminum cap to obtain an ihjectable preparation.

CLAIMS:

1. A cephalosporin compound of the following formula (I), or a pharmaceutically acceptable salt thereof

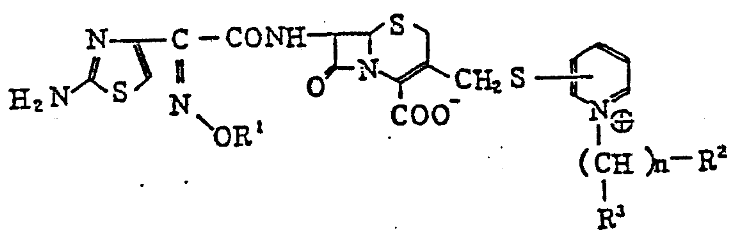

(I)

wherein $R^1$ represents a $C_1$ to $C_5$ alkyl group which may have at least one substituent selected from cyano and halogen moieties; $R^2$ represents a hydroxy $C_1$ to $C_5$ alkyl group, a cyano group, a $C_1$ to $C_5$ alkylthio group, a $C_2$ to $C_5$ alkenyl group substitutable by carboxy, a $C_2$ to $C_5$ alkinyl group, an imidazolyl group, a $C_3$ to $C_6$ alicyclic group, a 2-oxo-1,3-dioxol-5-yl group, a 2-carboxy-1,3-dithiolan-2-yl group, $-COR^A$ (wherein $R^A$ is an amino group, a pyridyl group, a carboxyl group, a $C_3$ to $C_6$ alicyclic group, or a $C_1$ to $C_5$ alkyl group which is unsubstituted or substituted by halogen), or

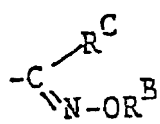

(wherein $R^B$ represents a $C_1$ to $C_5$ alkyl group which is unsubstituted or substituted by carboxyl, and $R^C$ represents a carboxyl group or a $C_1$ to $C_5$ alkyl group); n represents 1, 2 or 3; and $R^3$ represents a hydrogen atom or a $C_1$ to $C_5$ alkyl group.

2. A compound according to claim 1 wherein $R^1$ is a $C_1$ to $C_5$ alkyl group which may have at least one substituent selected from cyano and halogen moieties; $R^2$ represents $-COR^A$ (wherein $R^A$ is an amino group, a pyridyl group, a carboxyl group, a $C_3$ to $C_6$ alicyclic group, or a $C_1$ to $C_5$ alkyl group which is unsubstituted or substituted

by halogen), or 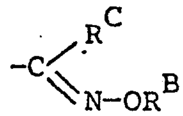 (wherein $R^B$ is a $C_1$ to $C_5$ alkyl group which is unsubstituted or substituted by carboxyl, and $R^C$ is a carboxyl group or a $C_1$ to $C_5$ alkyl group); or a salt thereof.

3. One or more compounds according to claim 1 selected from those of the following formulae and salts (e.g. nitrates and sulfates) thereof:

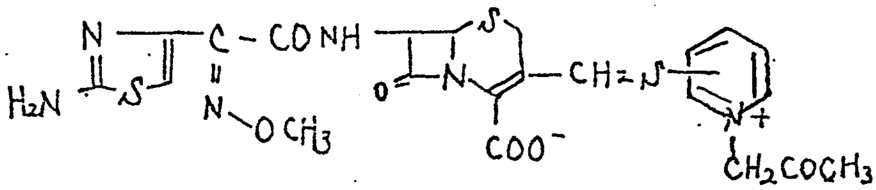

;

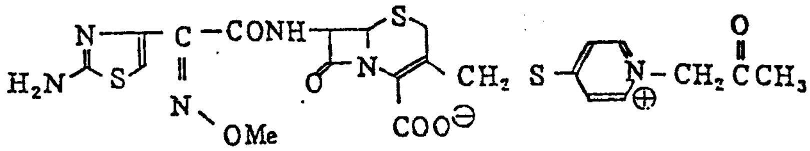

;

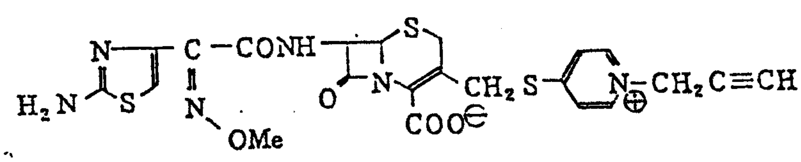

.

4. One or more compounds according to claim 1 selected from the following compounds and salts (e.g. nitrates and sulfates) thereof: (Z)-7-[α-(2-amino-4-thiazolyl)-α-(methoxyimino)acetamido]-3-[1-acetonyl-4-pyridinio]thiomethyl-$\Delta^3$-cephem-4-carboxylate;

(Z)-7-[α-(2-amino-4-thiazolyl)-α-(methoxyimino)acetamido]-3-[1-acetonyl-4-pyridinio]thiomethyl-Δ$^3$-cephem-4-carboxylate (inner salt);

(Z)-7-[α-(2-amino-4-thiazolyl)-α-(methoxyimino)acetamido]-3-(1-cyanomethyl-4-pyridinio)thiomethyl-Δ$^3$-cephem-4-carboxylate ;

(Z)-7-[α-(2-amino-4-thiazolyl)-α-(methoxyimino)acetamido]-3-[1-propargyl-4-pyridinio thiomethyl-Δ$^3$-cephem-4-carboxylate;

(Z)-7-[α-(2-amino-4-thiazolyl)-α-(2-fluoroethoxyimino)acetamido]--3-(1-acetonyl-4-pyridinio)thiomethyl-Δ$^3$-cephem-4-carboxylate;

(Z)-7-[α-(2-amino-4-thiazolyl)-α-(methoxyimino)acetamido]-3-[1-(4-imidazolyl)methyl-4-pyridinio]thiomethyl-Δ$^3$-cephem-4-carboxylate; and

(Z)-7-[α-(2-amino-4-thiazolyl)-α-(methoxyimino)acetamido]-3-[1-(2-oxopentyl)-4-pyridinio]thiomethyl-Δ$^3$-cepehm-4-carboxylate.

5. A compound of the following formula or a salt thereof

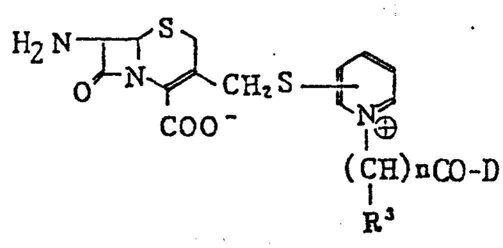

(wherein h is 1, 2 or 3, and D is $C_1$ to $C_5$ alkyl).

6. A method of preparing a compound of the following formula (I) or a salt thereof

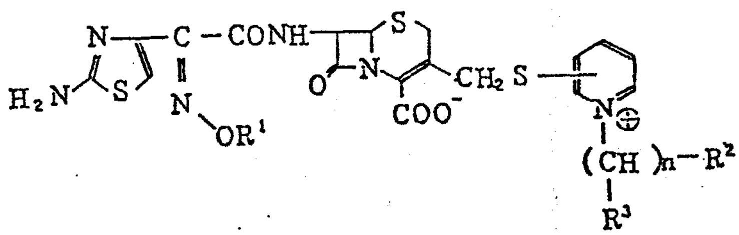

(I)

[wherein $R^1$ represents a $C_1$ to $C_5$ alkyl group which may have at least one substituent selected from cyano and halogen moieties; $R^2$ represents a hydroxy $C_1$ to $C_5$ alkyl group, a cyano group, a $C_1$ to $C_5$ alkylthio group, a $C_2$ to $C_5$ alkenyl group substitutable by carboxy, a $C_2$ to $C_5$ alkinyl group, an imidazolyl group, a $C_3$ to $C_6$ alicyclic group, a 2-oxo-1,3-dioxol-5-yl group, a 2-carboxy-1,3-dithiolan-2-yl group, $-COR^A$ (wherein $R^A$ is an amino group, a pyridyl group, a carboxyl group, a $C_3$ to $C_6$ alicyclic group, or a $C_1$ to $C_5$ alkyl group which is unsubstituted or substituted by halogen),or

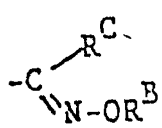 (wherein $R^B$ represents a $C_1$ to $C_5$ alkyl group which is unsubstituted or substituted by carboxyl, and $R^C$

represents a carboxyl group or a $C_1$ to$C_5$ alkyl group); n represents 1, 2 or 3; and $R^3$ represents a hydrogen atom or a $C_1$ to $C_5$ alkyl group

which comprises reacting a compound of the formula

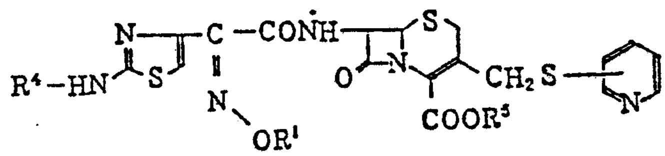

with a compound of the formula

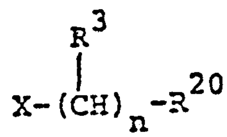

[wherein $R^4$ is a hydrogen atom or a protective group for an amino group; $R^5$ is a hydrogen atom or a protective group for a carboxyl group; $R^{20}$ is $R^2$ in which any carboxy group may be protected; and X is a halogen atom or $-OSO_2R^6$ (wherein $R^6$ is a $C_1$ to $C_5$ alkyl group, a trifluoromethyl group, or an aryl group); and wherein at least one reactant is optionally in salt form] and optionally releasing any protective group(s) and/or converting the product to or from salt form.

7. A method of preparing a compound of formula (I) as defined in claim 6 or a salt thereof which comprises reacting a compound of the formula

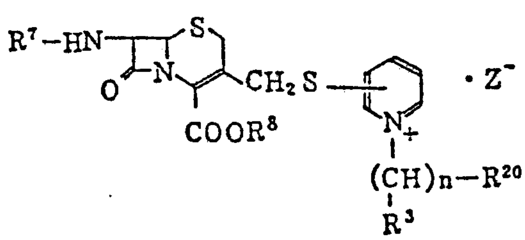

with a compound of the formula

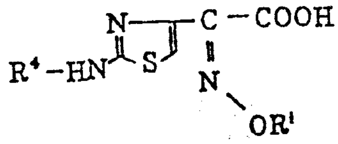

or a reactive derivative thereof [wherein $R^1$, $R^2$, $R^3$, $R^4$, $R^5$, $R^{20}$ and n are as defined in claim 6; $R^7$ is a hydrogen atom or an tri-($C_1$ to $C_5$ alkyl) silyl group; $Z^-$ represents an anion; and $R^8$ is a hydrogen atom or a protective group for a carboxyl group, except that $-COOR^8$ may be $-COO^-$ in which case $Z^-$ is absent; and wherein at least one reactant is optionally in salt form] and optionally releasing any protective group(s) and/or converting the product to or from salt form.

8. A method of preparing a compound of formula (I) as defined in claim 6 or a salt thereof which comprises reacting a compound of the formula

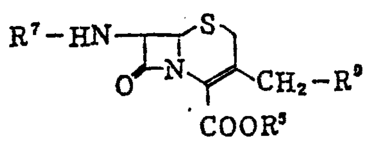

with a compound of the formula

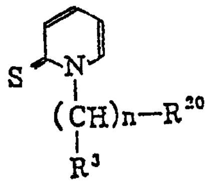
,
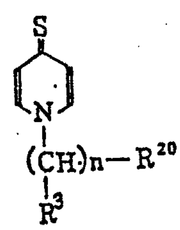
, or
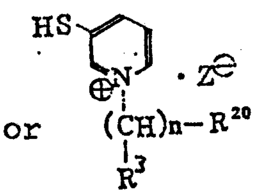

to form a compound of the formula.

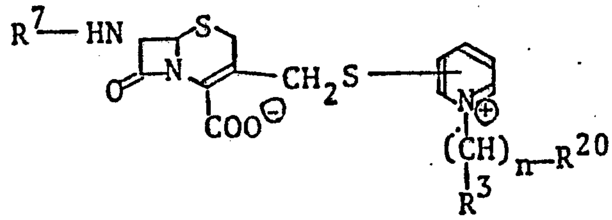

and then reacting the formed compound with a compound of the formula

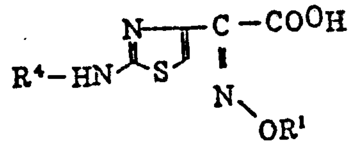

or a reactive derivative thereof [wherein $R^1$, $R^2$, $R^3$, $R^4$, $R^5$, $R^7$, $R^{20}$ and n are as defined in claims 6 and 7 and $R^9$ is a halogen atom or $C_1$ to $C_5$ acyloxy group; and wherein at least one reactant is optionally in salt form] and optionally releasing any protective group(s) and/or converting the product to or from salt form.

9. A method of preparing a compound of the following formula

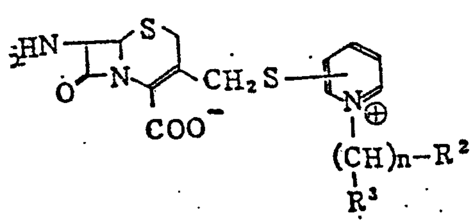

which comprises reacting a compound of the formula

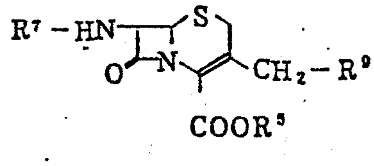

with a compound of the formula

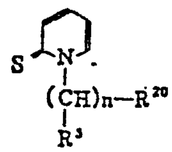 , 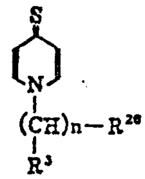 or 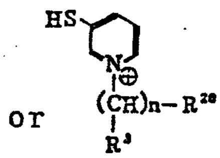

[wherein $R^2$, $R^3$, $R^5$, $R^7$, $R^9$, $R^{20}$ and n are as defined in claims 6 to 8] and, if necessary, releasing any protective group(s).

10. A method of preparing a compound of formula (I) as defined in claim 6 or a salt thereof which comprises reacting a compound of the formula

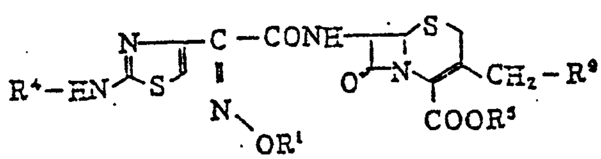

with a compound of the formula

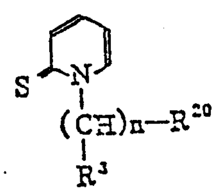 , 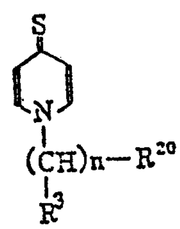 or 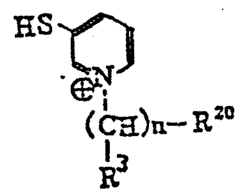

[wherein $R^1$, $R^2$. $R^3$, $R^4$, $R^5$, $R^9$, $R^{20}$ and n are as defined in claims 6 to 8; and wherein at least one reactant is optionally in salt form] and then optionally releasing any protective group(s) and/or converting the product to or from salt form.

11. An antibacterial pharmaceutical composition containing at least one compound according to any of claims 1 to 4 as active component.